(19) Europäisches Patentamt
European Patent Office
Office européen des brevets

(11) Publication number: **0 299 101 B1**

(12) **EUROPEAN PATENT SPECIFICATION**

(45) Date of publication of patent specification: **23.09.92**

(51) Int. Cl.5: **C07D 223/32**, C07D 495/04, A61K 31/55, //(C07D495/04, 333:00,223:00)

(21) Application number: **87110253.9**

(22) Date of filing: **16.07.87**

(54) **Fused benzazepines.**

(43) Date of publication of application:
**18.01.89 Bulletin 89/03**

(45) Publication of the grant of the patent:
**23.09.92 Bulletin 92/39**

(84) Designated Contracting States:
**ES GR**

(56) References cited:
**WO-A-87/04430**
**GB-A- 1 221 324**
**US-A- 3 393 192**

(73) Proprietor: **SCHERING CORPORATION**
**2000 Galloping Hill Road**
**Kenilworth New Jersey 07033(US)**

(72) Inventor: **Berger, Joel Gilbert**
**50 West Lindsley Road**
**Cedar Grove New Jersey 07009(US)**
Inventor: **Chang, Wei Kong**
**63 West Cedar Street**
**Livingston New Jersey 07039(US)**
Inventor: **Gold, Elijah Herman**
**10 Roosevelt Avenue**
**West Orange New Jersey 07052(US)**
Inventor: **Clader, John Welch**
**428 North Union Avenue**
**Cranford New Jersey 07016(US)**

(74) Representative: **von Kreisler, Alek, Dipl.-Chem. et al**
**Deichmannhaus am Hauptbahnhof**
**W-5000 Köln 1(DE)**

**EP 0 299 101 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to fused derivatives of compounds having a fused ring nucleus which includes a 2,3,4,5-tetrahydro-1H-3-benzazepine system, to methods for their preparation, to intermediates useful in their preparation, and to pharmaceutical compositions containing them. The compounds have valuable pharmaceutical properties in the treatment of psychoses, depression, pain and hypertension.

Substituted 1-phenyl-2,3,4,5-tetrahydro-1H-3-benzazepines have been described in the art. For example, see U.S. Patents 3,393,192, 3,609,138, 4,011,319, 4,284,555 and 4,477,378 as well as British Patent 1,118,688. The activities discussed for the compounds disclosed in these patents include anti-bacterial effects, central nervous system effects and hypotensive effects.

This invention relates to trans isomers of compounds according to the structural formula I, and pharmaceutically acceptable salts thereof,

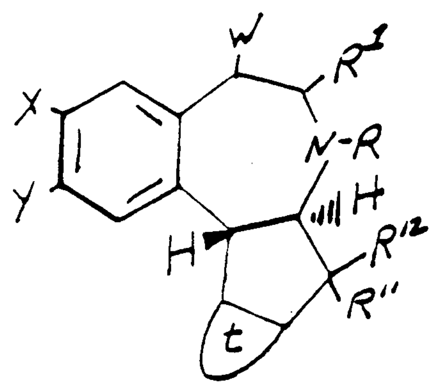

I

wherein:

R is hydrogen, alkyl, $-CH_2CH=CH_2$ or $-CH_2-$◁ ;

$R^1$, $R^{11}$ and $R^{12}$ may be the same or different and each is hydrogen or alkyl;

X is hydrogen, halo, alkyl, alkylthio, alkylsulfinyl, alkylsulfonyl, hydroxy, alkoxy or trifluoromethyl;

Y is hydrogen, hydroxy, alkoxy,

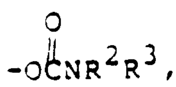, 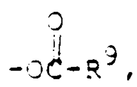, $-NR^1_2$, 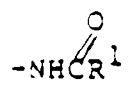 or 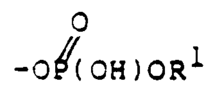

where $R^1$ is as defined above;

W is hydrogen, hydroxy or alkoxy;

ring (t) represents a fused thiophene or fused benzene ring, said fused benzene ring optionally being substituted with a substituent Z as defined below;

$R^2$ and $R^3$ are independently hydrogen (provided that both are not hydrogen), alkyl, aralkyl, cycloalkyl, aryl, hydroxyalkyl, or alkoxyalkyl;

in addition, when one of $R^2$ and $R^3$ is as defined above, the other may be $-R^4NR^5R^6$ {wherein $R^4$ is alkanediyl, $R^5$ is hydrogen or alkyl and $R^6$ is alkyl, or $R^5$ and $R^6$ together with the nitrogen atom form a 1-azetidinyl, 1-pyrrolidinyl, 1-piperidinyl, 1-(4-alkylpiperazinyl), 4-morpholinyl or 1-(hexahydroazepinyl) group};

in further addition, $R^2$ and $R^3$ together with the nitrogen atom may form a 1-azetidinyl, 1-pyrrolidinyl, 1-piperidinyl, 4-morpholinyl, 1-(4-alkylpiperazinyl), 1-(4-alkoxyalkylpiperazinyl), 1-(4-hydroxyalkylpiperazinyl), 1-(3-hydroxyazetidinyl), 1-(3-alkoxyazetidinyl), 1-(3-hydroxypyrrolidinyl), 1-(3-alkoxypyrrolidinyl), 1-(3- or 4-hydroxypiperidinyl), 1-(3- or 4-alkoxypiperidinyl), 1-(4-oxopiperidinyl) or 1-(3-oxopyrrolidinyl) ring;

in still further addition, when $R^2$ is hydrogen, $R^3$ may be $-CHR^7CO_2R^8$, wherein $R^7$ and $R^8$ are independently hydrogen, alkyl or aralkyl;

$R^9$ is alkyl, aralkyl, aryl, alkoxyalkyl, aryloxyalkyl, aralkoxyalkyl, cycloalkylalkyl, alkoxycarbonylalkyl, cycloalkyl, 1-adamantyl, cycloalkoxyalkyl, alkoxy, aralkoxy, cycloalkoxy, aryloxy or $-CHR^7NHR^8$ {wherein $R^7$ and $R^8$ are as defined above}; and

Z is X as defined above, amino, alkylamino or

O
$-NHCR^{10}$

{wherein $R^{10}$ is hydrogen, alkyl or aryl}.

A preferred subgenus is represented by structural formula I above, where all of the substituents are as defined above but with the provision that when X is $OCH_3$, Y is OH, Z and $R^1$ are both H, R cannot be $CH_3$.

Another preferred subgenus is represented by structural formula Ia below:

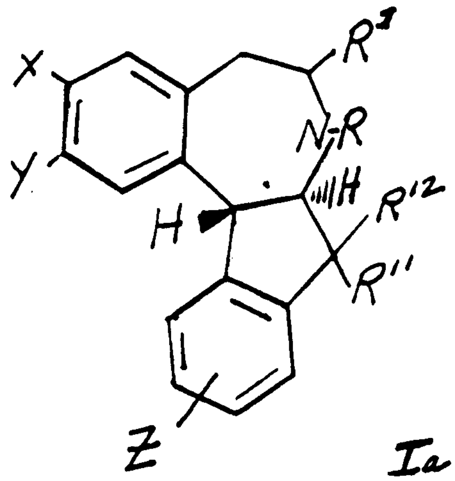

Ia

wherein R, $R^1$, $R^{11}$, $R^{12}$, X, Y, and Z, are as defined above.

A third preferred subgenus of formula I is wherein Y is

O
$-O-CNR^2R^3$

(wherein $R^2$ and $R^3$ are both alkyl or one of $R^2$ and $R^3$ is hydrogen and the other is alkyl), $-NHR^1$ (wherein $R^1$ is hydrogen or methyl),

O
$-NHCR^1$

(wherein $R^1$ is hydrogen or methyl),

O
$-OCR^9$

(wherein $R^9$ is as defined above) amino or hydroxy. W is preferably H. X is hydrogen, alkyl, halogen or alkoxy; while Z is hydrogen, halogen, alkyl, hydroxy or alkoxy. R is methyl and $R^1$ is hydrogen or methyl. Ring (t) represents a fused benzene ring optionally substituted with halo, alkyl, or $-OR^1$.

A third preferred subgenus of compounds is those of formula Ia above wherein R is methyl; $R^1$, $R^{11}$ and $R^{12}$ are hydrogen; X is hydrogen, methyl, methoxy, chloro or bromo; Y is hydroxy, amino,

O
$-OCR^9$

(wherein $R^9$ is defined as above,

$$-O\overset{O}{\overset{\|}{C}}N(CH_3)_2$$

or $-NHCH_3$; and Z is hydrogen, halo, alkyl or $-OR^1$ (wherein $R^1$ is hydrogen or alkyl); or a pharmaceutically acceptable salt of such a compound.

Preferred compounds of formula I include

(1) trans-5,6,7,7a,8,12b-hexahydro-2-hydroxy-3-chloro-7-methyl-benz(d)indeno-[2,1-b]azepine or a pharmaceutically acceptable salt thereof;

(2) (+)-trans-5,6,7,7a,8,12b-hexahydro-2-hydroxy-3-chloro-7-methyl-benz(d)-indeno[2,1-b]azepine or a pharmaceutically acceptable salt thereof;

(3) (-)-trans-5,6,7,7a,8,12b-hexahydro-2-hydroxy-3-chloro-7-methyl-benz[d]-indeno[2,1-b]azepine or a pharmaceutically acceptable salt thereof;

(4) trans-5,6,7,7a,8,12b-hexahydro-2-hydroxy-3-methoxy-7-methyl-benz[d]-indeno[2,1-b]azepine or a pharmaceutically acceptable salt thereof;

(5) trans - 5,6,7,7a,8,12b - hexahydro-2-amino-3-chloro-7-methyl-benz[d]-indeno[2,1-b]azepine or a pharmaceutically acceptable salt thereof;

(6) trans - 5,6,7,7a,8,12b-hexahydro-2-hydroxy-7-methyl-benz[d]-indeno[2,1-b]azepine or a pharmaceutically acceptable salt thereof;

(7) trans - 5,6,7,7a,8,12b-hexahydro-3,7-dimethyl,2-hydroxy-benz[d]-indeno[2,1-b]azepine or a pharmaceutically acceptable salt thereof;

(8) trans - 5,6,7,7a,8,12b-hexahydro-3-chloro-7-cyclo-propylmethyl-2-hydroxy-benz[d]indeno[2,1-b]-azepine or a pharmaceutically acceptable salt thereof;

(9) trans - 5,6,7,7a,8,12b-hexahydro-7-allyl-3-chloro-2-hydroxy-benz[d]indeno[2,1-b]azepine or a pharmaceutically acceptable salt thereof;

(10) trans - 5,6,7,7a,8,12b-hexahydro-3-chloro-2-hydroxy-7,8,8-trimethyl-benz[d]-indeno[2,1-b]azepine or a pharmaceutically acceptable salt thereof; and

(11) trans-3-chloro-5,6,7,7a,8,11b-hexahydro-7-methyl-thieno[2',3':4,5]cyclopenta[1,2-a][3]benzazepine-2-ol or a pharmaceutically acceptable salt thereof.

Particularly preferred compounds are:

(1) trans 5,6,7,7a,8,12b-hexahydro-2-hydroxy-3-chloro-7-methyl-benz-[d]indeno-[2,1-b]azepine or a pharmaceutically acceptable salt thereof;

(2) (+) - trans-5,6,7,7a,8,12b-hexahydro-2-hydroxy-3-chloro-7-methyl-benz[d]-indeno[2,1-b]azepine or a pharmaceutically acceptable salt thereof;

(3) (-) trans -5,6,7,7a,8,12b-hexahydro-2-hydroxy-3-chloro-7-methyl-benz[d]-indeno[2,1-b]azepine or a pharmaceutically acceptable salt thereof;

(4) trans - 5,6,7,7a,8,12b-hexahydro-2-hydroxy-3-methoxy-7-methyl-benz[d]-indeno[2,1-b] azepine or a pharmaceutically acceptable salt thereof;

(5) trans - 5,6,7,7a,8,12b-hexahydro-2-amino-3-chloro-7-methyl-benz[d]-indeno[2,1-b]azepine or a pharmaceutically acceptable salt thereof;

(6) trans - 5,6,7,7a,8,12b-hexahydro-2-hydroxy-7-methyl-benz[d]-indeno[2,1-b]azepine or a pharmaceutically acceptable salt thereof;

(7) trans - 5,6,7,7a,8,12b-hexahydro-3,7-dimethyl,2-hydroxy-benz[d]-indeno[2,1-b]azepine or a pharmaceutically acceptable salt thereof; and

(8) trans - 5,6,7,7a,8,12b-hexahydro-2-hydroxy-3-chloro-7,8,8-tri-methyl-benz[d]indeno[2,1-b]azepine.

Another aspect of the invention is the use of a compound of formula I for the preparation of a pharmaceutical composition useful in the treatment of psychoses, pain and/or depression.

Yet another aspect of the invention comprises a method of making a pharmaceutical composition comprising mixing a compound of formula I with a pharmaceutically acceptable carrier.

Still another aspect of the invention comprises intermediate compounds having the formulae II, XI, XIII and XIV

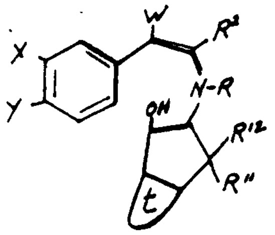

X

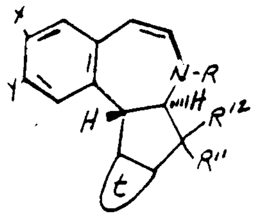

XI

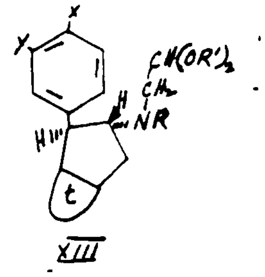

XIII

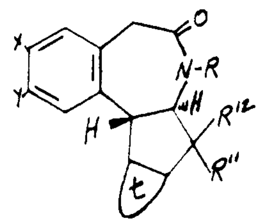

XIV

useful in the preparation of compounds of formula I wherein:

R is hydrogen, alkyl, $-CH_2CH=CH_2$ or $CH_2$-◁ ;

$R^1$, $R^{11}$ and $R^{12}$ are the same or different and each is hydrogen or alkyl;

X is hydrogen, halo, alkyl, alkylthio, alkylsulfinyl, alkylsulfonyl, hydroxy, alkoxy or trifluoromethyl;

Y is hydrogen, hydroxy, alkoxy,

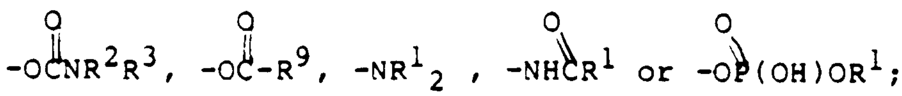

W is hydrogen, hydroxy or alkoxy;

ring (t) represents a fused thiophene or fused benzene ring, said fused benzene ring optionally being substituted with a substitutent Z as defined below;

$R^2$ and $R^3$ are independently hydrogen (provided that both are not hydrogen), alkyl, aralkyl, cycloalkyl, aryl, hydroxyalkyl, or alkoxyalkyl;

in addition, when one of $R^2$ and $R^3$ is as defined above, the other may be $-R^4NR^5R^6$ {wherein $R^4$ is alkanediyl, $R^5$ is hydrogen or alkyl and $R^6$ is alkyl, or $R^5$ and $R^6$ together with the nitrogen atom form a 1-azetidinyl, 1-pyrrolidinyl, 1-piperidinyl, 1-(4-alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-alkylpiperazinyl), 1-(4-alkoxyalkylpiperazinyl), 1-(4-hydroxyalkylpiperazinyl), 1-(3-hydroxyazetidinyl), 1-(3-alkoxyazetidinyl), 1-(3-hydroxypyrrolidinyl), 1-(3-alkoxypyrrolidinyl), 1-(3- or 4-hydroxypiperidinyl), 1-(3- or 4-alkoxypiperidinyl), 1-(4-oxopiperidinyl) or 1-(3-oxopyrrolidinyl) ring;

in still further addition, when $R^2$ is hydrogen, $R^3$ may be $-CHR^7CO_2R^8$, wherein $R^7$ and $R^8$ are independently hydrogen, alkyl or aralkyl;

$R^9$ is alkyl, aralkyl, aryl, alkoxyalkyl, aryloxyalkyl, aralkoxyalkyl, cycloalkylalkyl, alkoxycarbonylalkyl, cycloalkyl, 1-adamantyl, cycloalkoxyalkyl, alkoxy, aralkoxy, cycloalkoxy, aryloxy or $-CHR^7NHR^8$ {wherein $R^7$ and $R^8$ are as defined above};

R′ is a $C_1$-$C_3$ alkyl; and

Z is X as defined above, amino, alkylamino or

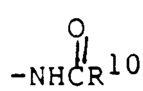

{wherein $R^{10}$ is hydrogen, alkyl or aryl}.

Another aspect of the invention is a process for producing a compound having the structural formula I

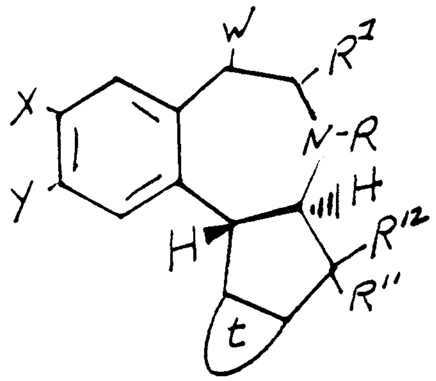

I

wherein:

R is hydrogen, alkyl, $-CH_2CH=CH_2$ or $-CH_2$-◁ ;

$R^1$, $R^{11}$ and $R^{12}$ are the same or different and each is hydrogen or alkyl;

X is hydrogen, halo, alkyl, alkylthio, alkylsulfinyl, alkylsulfonyl, hydroxy, alkoxy or trifluoromethyl;

Y is hydrogen, hydroxy, alkoxy,

$$-O\overset{O}{\overset{\|}{C}}NR^2R^3,\ -O\overset{O}{\overset{\|}{C}}-R^9,\ -NR^1_2\ ,\ -NH\overset{O}{\overset{\|}{C}}R^1 \text{ or } -O\overset{O}{\overset{\|}{P}}(OH)OR^1;$$

W is hydrogen, hydroxy or alkoxy;

ring (t) represents a fused thiophene or fused benzene ring said fused benzene ring optionally being substituted with a substitutent Z as defined below;

$R^2$ and $R^3$ are independently hydrogen (provided that both are not hydrogen), alkyl, aralkyl, cycloalkyl, aryl, hydroxyalkyl, or alkoxyalkyl;

in addition, when one of $R^2$ and $R^3$ is as defined above, the other may be $-R^4NR^5R^6$ {wherein $R^4$ is alkanediyl, $R^5$ is hydrogen or alkyl and $R^6$ is alkyl, or $R^5$ and $R^6$ together with the nitrogen atom form a 1-azetidinyl, 1-pyrrolidinyl, 1-piperidinyl, 1-(4-alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-alkylpiperazinyl), 1-(4-alkoxyalkylpiperazinyl), 1-(4-hydroxyalkylpiperazinyl), 1-(3-hydroxyazetidinyl), 1-(3-alkoxyazetidinyl), 1-(3-hydroxypyrrolidinyl), 1-(3-alkoxypyrrolidinyl), 1-(3- or 4-hydroxypiperidinyl), 1-(3- or 4-alkoxypiperidinyl), 1-(4-oxopiperidinyl) or 1-(3-oxopyrrolidinyl) ring;

in still further addition, when $R^2$ is hydrogen, $R^3$ may be $-CHR^7CO_2R^8$, wherein $R^7$ and $R^8$ are independently hydrogen, alkyl or aralkyl;

$R^9$ is alkyl, aralkyl, aryl, alkoxyalkyl, aryloxyalkyl, aralkoxyalkyl, cycloalkylalkyl, alkoxycarbonylalkyl, cycloalkyl, 1-adamantyl, cycloalkoxyalkyl, alkoxy, aralkoxy, cycloalkoxy, aryloxy or $-CHR^7NHR^8$ {wherein $R^7$ and $R^8$ are as defined above}; and

Z is X as defined above, amino, alkylamino or

$$-NH\overset{O}{\overset{\|}{C}}R^{10}$$

{wherein $R^{10}$ is hydrogen, alkyl or aryl} characterized by:

(A) intramolecular condensation of a compound of the formula

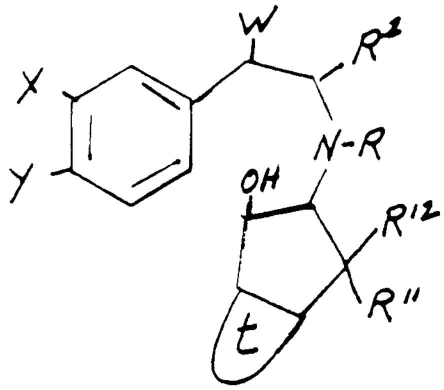

II

Compounds according to formula I may be prepared by intramolecular condensation of a compound having the structural formula II in the presence of a dehydration catalyst. Effective dehydration catalysts include sulfuric acid, methanesulfonic acid, trifluoromethanesulfonic acid, phosphoric acid and anhydrous hydrofluoric acid.

The intramolecular condensation described above may be performed at various temperatures and pressures, e.g., between 0 ° C and 100 ° C and at reduced, atmospheric or elevated pressure. An inert solvent may be employed or the reaction may be run in the absence of solvent. The time required for obtaining the desired product varies somewhat with the temperature, pressure and batch size but the reaction is generally complete within 24 hrs.

i. Compounds of formula II may be obtained by reacting an amine of formula V with a compound of formula VI

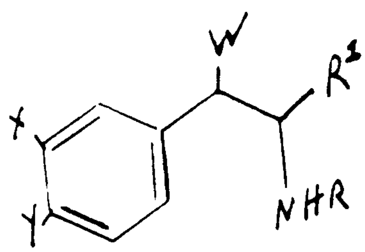

V

+

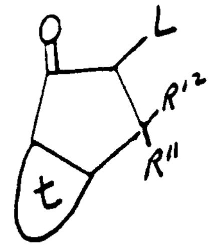

VI

to produce a compound of formula VII

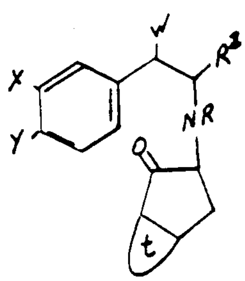

VII

followed by reduction of the carbonyl group in the product of formula VII to hydroxy. The symbol L represents a readily displaceable moiety commonly known as a "leaving group". Suitable leaving groups used by those skilled in the art include but are not limited to the halides,. e.g., chlorine, bromine or

iodine, and $OSO_2R$ wherein R may be hydrogen, alkyl, perfluoroalkyl, arylalkyl, or aryl (for example para-toluenesulfonyl). Preferred reducing agents for the reduction step include $NaBH_4$, $LiAlH_4$, $BH_3$, and $NaAlH_2(OCH_2CH_2OCH_3)_2$. Catalytic reductions using catalysts such as palladium on carbon or Raney nickel and hydrogen gas at 1 to 10 atmospheres pressure are also effective.

ii. Compounds of formula II may also be obtained by reacting an amine of formula VIII with a compound of formula IX under the conditions described in the preceding paragraph but without the reduction step:

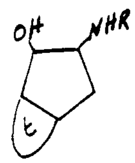

VIII

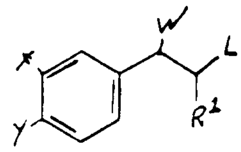

IX

The amine of formula VIII may be made by methods known by those skilled in the art including J. Org. Chem. 52, 1649(1987).

iii Compounds of formula II can be prepared by reacting an aldehyde ($R^1$ = H) or ketone ($R^1$ = alkyl) of formula X with an amino alcohol of formula VIII in the presence of an appropriate reducing agent such as sodium cyanoborohydride at pH 4-7.

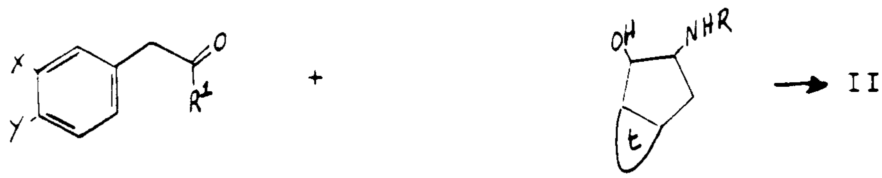

X VIII

iv. Compounds of formula II may also be prepared by reaction of compounds of formula VIII with aldehydes or ketones of formula X with prior removal of water of condensation followed by reduction of the resulting intermediate condensation product with reducing agents such as $NaBH_4$ or $NaCNBH_3$, or by catalytic reduction using catalysts such as palladium on carbon or Raney nickel under a hydrogen atmosphere at 1-10 atmospheres pressure.

(B) Compounds of formula I also may be prepared by reacting a compound of the formula

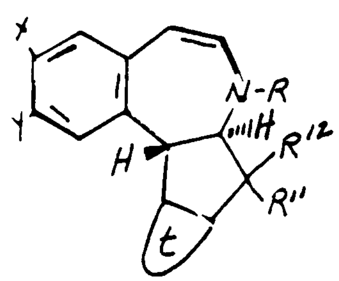

XI

with a suitable reducing agent to produce the compound of formula I. Preferred reducing agents comprise hydrogen and a catalyst with $PtO_2$ being a particularly preferred catalyst, and $NaCNBH_3$ at pH4-7, preferably in the presence of acetic acid.

The compound of formula XI may be prepared, for example, by reacting a compound of formula XII same as XXI with a 1-halo-2,2-dialkoxyethane in the presence of a suitable solvent such as dimethylformamide and a catalyst such as an alkali metal iodide, preferably potassium iodide, to produce a compound of formula XIII, where R′ is a $C_1$-$C_3$ alkyl

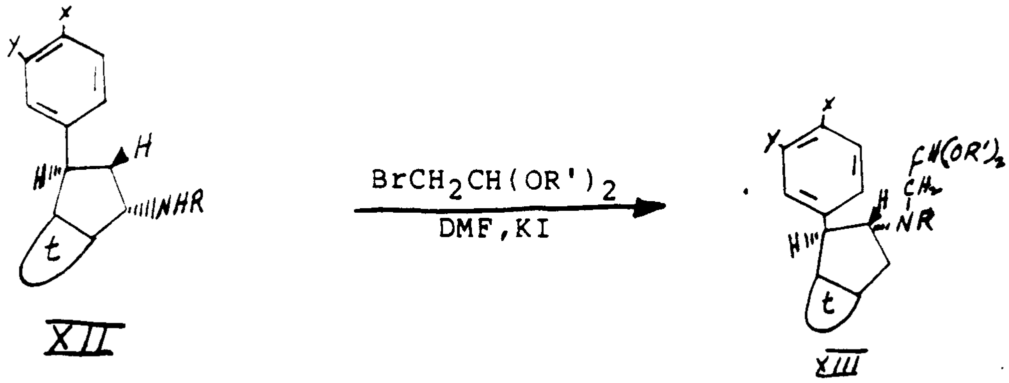


XII XIII

The compound of formula XIII may be reacted with a strong acid such as trifluoromethane sulfonic acid, methanesulfonic acid, or sulfuric acid at a temperature ranging between about 0-25 °C, to produce the compound of formula XI and in addition compounds of formula I wherein W is OH or OR'.

C. Compounds of formula I also may be prepared by reacting a compound of formula XIV

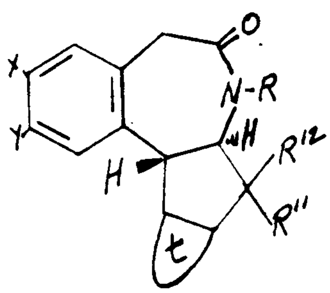

XIV

with a reducing agent such as $LiAlH_4$ or $BH_3$, preferably $BH_3$, at a temperature ranging between about 0 °C and about 70 °C to produce the compound of formula I.

One method for preparing the compound of formula XIV is set forth below. This method is particularly applicable where Y is OH or alkoxy. Where Y is one of the other substituents defined, additional process steps known in the art may be necessary in addition to those described below. Compound XIV may be prepared by first reacting a compound of formula XV with a compound of XVa

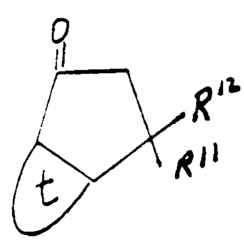

+

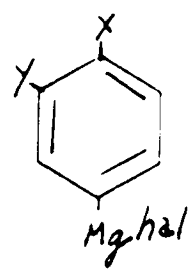

XV XVa

to produce a compound of the formula XVI

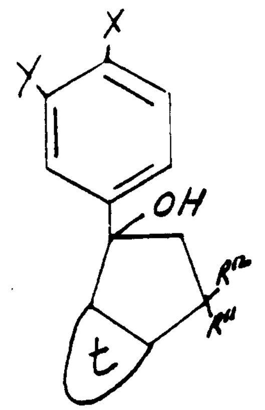

XVI

which may be dehydrated to a compound of formula XVII using acidic catalysts such as toluene sulfonic acid or phosphorus oxychloride/pyridine at a temperature ranging between about 20 ° C and about 120 ° C with continuous removal of water.

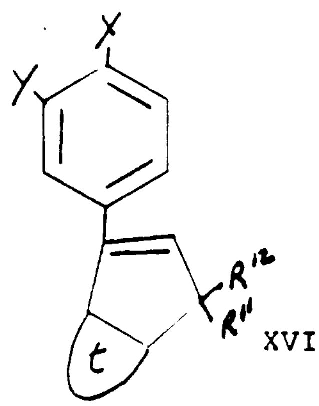

XVII

The compound of formula XVII may be oxidized to produce a compound of formula XVIII below using m-chloroperbenzoic acid at a temperature ranging between about 0 ° C and about 20 ° C, followed by contact with an alkali metal hydroxide such as sodium hydroxide, followed by contact with a strong mineral acid.

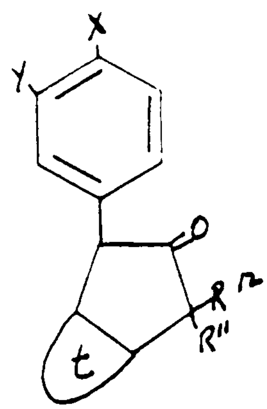

XVIII

The compound of formula XVIII may in turn be reacted with an alkyl amine with continuous removal of water to produce a compound of formula XIX

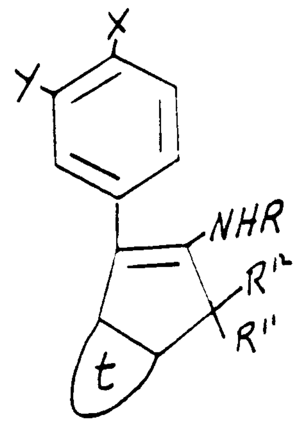

XIX

which then may be reduced in the presence of a catalyst such as zinc dust and acetic acid or $NaCNBH_3$ at pH 4-7, preferably in the presence of acetic acid or sodium cyanoforohydride in the presence of acetic acid to produce a compound of formula XX

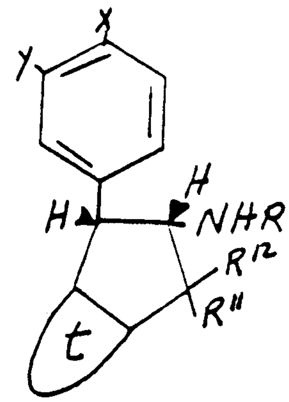

XX

which may then be treated with a strong base in dimethylsulfoxide (DMSO) or a mixed solvent comprising DMSO plus a polar aprotic solvent, e.g., dimethylformamide (DMF) to produce a compound of formula XXI. Strong bases utilized are preferably potassium tert butoxide or sodium hydride.

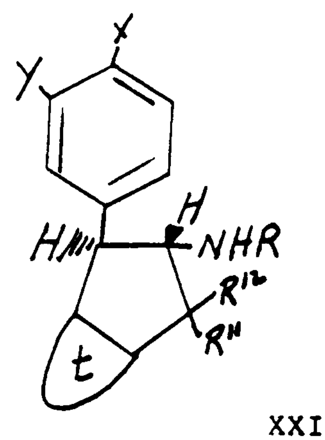

XXI

Alternatively, the compound of formula XVII may be reacted with $BH_3$-methyl sulfide complex in a halocarbon solvent (e.g. $CH_2Cl_2$) at temperature of 20°-65°, and the resulting reaction mixture treated with hydroxylamine-0-sulfonic acid at temperatures of 80-120° in a mixed solvent system such as diglyme/$CH_2Cl_2$ to furnish compounds of formula XXI with R = H.

The compound of formula XXI may be contacted with a haloacetyl halide to produce a compound of formula XXII

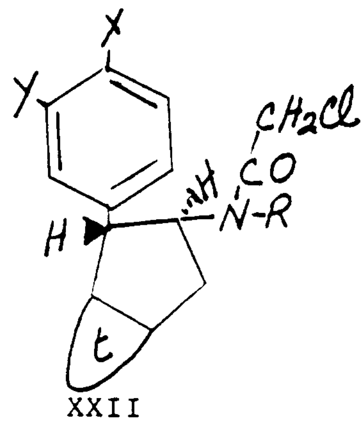

XXII

which may be exposed to light to produce the compound of formula XIV

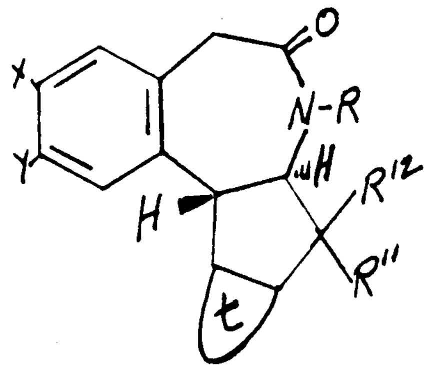

XIV

which may then be reduced to the compound of formula I.

In the above processes A-C, it is sometimes desirable and/or necessary to protect certain R, $R^1$, $R^{11}$, $R^{12}$, W, X, Y and Z groups during the reactions. Conventional protecting groups are operable. For example, the groups listed in column 1 of the following table may be protected as indicated in column 2 of the table:

| 1. Group to be Protected | 2. Protected Group |
|---|---|
| $-COOH$ | $-COO$alkyl, $-COO$benzyl, $-COO$phenyl |
| $>NH$ | $>N-CO_2$alkyl, $>N-CO_2$benzyl, $>N-CO_2CH_2CCl_3$ |
| $>CO$ | 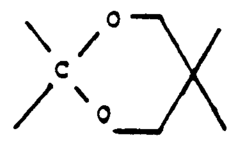 , 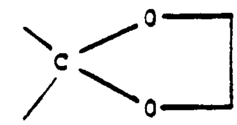 |
| $-OH$ | 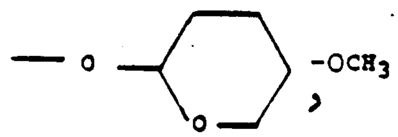 |
| $-NH_2$ | 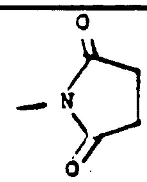 |

Of course, other protecting groups well known in the art may be used. After the reaction or reactions, the protecting groups may be removed by standard procedures.

Also, R, $R^1$, $R^{11}$, $R^{12}$, W, X, Y and Z groups in formula I may be varied by appropriate selection of starting materials from which the compounds are synthesized or by reacting a compound of formula I with a suitable reagent to effect the desired conversion of the substituent to another R, $R^1$, $R^{11}$, $R^{12}$, W, X, Y and Z group. The latter procedure is particularly applicable for changing the substituents X. For example, a chlorine substituent may be added in place of hydrogen by reaction with a chlorinating agent such as sulfuryl chloride in a non-reactive solvent. A hydroxymethyl substituent in the X position may be added in place of hydrogen by reaction with formaldehyde in a suitable solvent system, e.g., in a mixed solvent system consisting of dimethyoxyethane and aqueous potassium hydroxide, preferably at an elevated temperature. Such a hydroxymethyl substituent may be reduced to a methyl group by reaction with a catalyst such as palladium hydroxide in a hydrogen atmosphere under pressure. Methoxy substituents may be converted to hydroxy, e.g., by refluxing in a mixture of sodium hydride, DMF and ethanethiol, or by reaction with concentrated hydrobromic acid. Other substitutions may be accomplished using standard techniques.

When utilized herein and in the appended claims, the following terms, unless otherwise specified, have the following scope:

halo - represents fluoro, chloro, bromo or iodo;

alkyl (including, for example, the alkyl portions of alkylthio, alkoxy, aralkyl, alkoxyalkoxy, etc.) - represents straight or branched carbon chains having 1 to 6 carbon atoms;

cycloalkyl groups (including the cycloalkyl portion in cycloalkoxy groups) - represents saturated carbocyclic rings having 3 to 7 carbon atoms;

alkanediyl - represents a divalent, straight or branched hydrocarbon chain having from 1 to 6 carbon atoms, the two available bonds being from the same or different carbon atoms thereof, e.g., methylene, ethylene, ethylidene, $-CH_2CH_2CH_2-$,

$-CH_2CHCH_3$,

$=CHCH_2CH_3$, etc.;

aryl (including, for example, the aryl moiety in aralkyl or aralkoxy groups) - represents unsubstituted phenyl and phenyl mono substituted by alkyl, hydroxy, alkoxy, halo or trifluoromethyl.

The compounds of formula I possess analgesic, anticholinergic, antiaggressive and general tranquilizing properties. The invention therefore includes pharmaceutical compositions comprising a compound of formula I in combination with a pharmaceutically acceptable carrier and methods for treating mental disorders including psychoses, schizophrenia or depression in a mammal, or for the control of pain or anxiety in a mammal by administering an effective amount of a compound of formula I to the affected mammals. The compounds of formula I provide a long duration of activity.

Certain compounds of formula I wherein X and Y are hydroxy and R is hydrogen are also active as renal vasodilators. These compounds can thus be used in pharmaceutical compositions in combination with a pharmaceutically acceptable carrier and in methods for controlling hypertension by administering to a mammal a renal vasodilating effective amount of such a compound.

While the present invention is directed at trans isomers of formula I, racemic mixtures also would be useful. Therefore, it is not believed necessary to separate the trans compounds of formula I from the racemic mixture. All such isomeric forms and mixtures thereof are within the scope of the present invention. Unless otherwise indicated, the methods of preparation disclosed herein result in product distributions which include all possible structural isomers, although it is understood that physiological response may vary according to stereochemical structure. The isomers may be separated by conventional means such as fractional crystallization or HPLC.

Ring (t) may represent a fused thiophene ring. The sulfur atom in such fused thiophene ring may be in any of the non-fused positions of said ring.

Compounds of formulas I and Ia can exist in unsolvated as well as solvated forms, including hydrated forms. In general, the solvated forms, with pharmaceutically acceptable solvents such as water, ethanol and the like, are equivalent to the unsolvated forms for purposes of this invention.

The compounds of formulas I and Ia may form pharmaceutically acceptable salts with organic and inorganic acids. Examples of suitable acids for salt formation are hydrochloric, sulfuric, phosphoric, acetic, citric, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The free base forms differ from their respective salt forms somewhat in certain physical properties, such as solubility in polar solvents, but the salts are otherwise equivalent to their respective free base forms for purposes of the invention.

The compounds of formula I display pharmacological activity in test procedures designed to indicate anti-psychotic and anti-depressive activity. The compounds are non-toxic at pharmaceutically therapeutic doses.

## COMPETITIVE INHIBITION ASSAY

Many compounds capable of effecting reproducible physiological changes in neural tissues are believed to operate by binding at one or more receptor sites. Compounds which interact strongly with these receptor sites in *in vitro* tests, using homogenates of the target organ or structure, are expected to exhibit similar properties when administered *in vivo* and are, therefore, candidates for continued study as potential therapeutic and/or diagnostic agents.

Binding of a compound to a receptor site, *in vitro* is demonstrated by the specificity of binding and the saturability of the available sites. A methodology for characterization of D-1 and D-2 receptor binding and an interpretation of the data are described by Billard et al., *Life Sciences* 35, 1885 (1984) in which the binding of the benzazepine (R)-(+)-8-chloro-2,3,4,5-tetrahydro-3-methyl-5-phenyl-1H-3-benzazepin-7-ol hemimaleate (SCH 23390) to the dopamine D-1 receptor is characterized. A selectivity for D-1 receptor binding as compared to D-2 receptor binding is believed to confer the therapeutic advantage of avoiding troublesome and potentially irreversible neurological side effects associated with D-2 receptor occupancy.

## Materials and Methods

Tritiated SCH 23390 and tritiated spiperone (a potent D-2 receptor ligand) were obtained as described in the Billard et al. reference *supra* and serially diluted in 0.05 M Tris buffer, pH 7.4, as required. Compounds of this invention were synthesized as disclosed herein and diluted in 0.05 M Tris buffer, pH 7.4,

as required.

Tissue Preparation

Male Sprague-Dawley rats (200 to 250 g) from Charles River Breeding Laboratories, Mass. were used to obtain brain tissue. The rats were humanely sacrificed and their brains removed and placed on ice. Striatal tissue was excised, pooled, and homogenized (Brinkman Polytron, 10 sec) in 100 volumes (w/v) of ice cold 50 mM Tris buffer, pH 7.4 (at 25 °C). The homogenate was centrifuged at 20,000 ×g for 10 min. The resultant pellet was rehomogenized in Tris buffer and centrifuged again. The final pellet was resuspended in 50 mM Tris buffer pH 7.4 containing 120 mM NaCl, 5 mM KCl, 2 mM $CaCl_2$, and 1 mM $MgCl_2$.

Assay

Polypropylene incubation tubes received 100 $\mu$l of the individual test compounds at various concentrations dissolved or suspended in 0.05 M Tris, pH 7.4 containing 4 mg/ml methylcellulose, 100 $\mu$l of a solution of $^3$H-SCH 23390 in Tris buffer (final reaction mixture concentration =0.3 nM) or 100 $\mu$l of a solution of $^3$H-spiperone in Tris buffer (final concentration =0.2 nM) and 800 $\mu$l of tissue suspension (ca. 3 mg/assay). Tubes were incubated at 37 °C for 15 minutes and rapidly vacuum filtered through Whatman GF/B filters and rinsed 4 times with 4 ml of ice cold 50 mM Tris buffer, pH 7.4. The filters were transferred to scintillation vials, equilibated with 10 ml of scintillant (Scintosol, Isolab, Inc.) for 16 hours at 25 °C and the radioactivity determined in a liquid scintillation counter. $K_i$ values were determined as described by Billard et al. using the relationship $K_i = IC_{50}/(1 + ([L]/K_D))$ wherein $IC_{50}$ = concentration of test drug necessary to displace 50% of specifically bound $^3$H-Sch 23390, [L] = concentration of radioligand used in the assay, and $K_D$ = dissociation constant.

Results

The inhibition constants ($K_i$) determined from the assays for a series of compounds of the invention are as shown in Table I below.

TABLE I

| X | Y | W | $R^{11}$ | $R^{12}$ | R | $R^1$ | ring 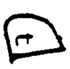 | Ki nM 23390 | Ki nM Spiperone |
|---|---|---|---|---|---|---|---|---|---|
| Cl | OH | H | H | H | $CH_3$ | H | 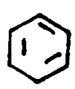 | 7 | 12000 |
| Cl | OH | H | H | H | H | H | 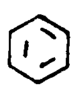 | 7 | 1580 |
| $OCH_3$ | OH | H | H | H | $CH_3$ | H. | 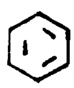 | | |

The comparatively small $K_i$ values of these compounds in the competitive binding assay with SCH 23390 indicate that the compounds of formula I bind strongly to the D-1 receptor site. The relatively high $K_i$ values for the D-2 site, for which spiperone is highly selective, indicate that the compounds are not specifically bound to that receptor site.

For preparing pharmaceutical compositions from the compounds of formula I, inert, pharmaceutically

acceptable carriers are admixed with the active compounds. The pharmaceutically acceptable carriers may be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents; it may also be an encapsulating material.

Liquid form preparations include solutions, suspensions and emulsions. As an example may be mentioned water or water-propylene glycol solutions for parenteral injection.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit.

The invention also contemplates alternative delivery systems including, but not necessarily limited to, transdermal delivery. The transdermal compositions can take the form of creams, lotions and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active components. The unit dosage form can be a packaged preparation, the package containing discrete quantities of preparation such as packeted tablets, capsules and powders in vials or ampules. The unit dosage form can also be a capsule, cachet or tablet itself, or it may be the appropriate number of any of these in a packaged form.

The quantity of active compound in a unit dose preparation may be varied or adjusted from 1 mg to 100 mg according to the particular application and the potency of the active ingredient and the intended treatment. This would correspond to a dose of about 0.02 to about 2.0 mg/kg which may be divided over 1 to 3 administrations per day. The composition may, if desired, also contain other therapeutic agents.

The dosages may be varied depending on the requirement of the patient, the severity of the condition being treating and the particular compound being employed. Determination of the proper dosage for a particular situation is within the skill of those in the medical art. For convenience, the total daily dosage may be divided and administered in portions throughout the day or by means providing continuous delivery.

The invention disclosed herein is exemplified by the following preparative examples which should not be construed to limit the scope of the disclosure. Alternative mechanistic pathways and analogous structures may be apparent to those skilled in the art.

Trans-6-Methyl-5,6,7,7a,8,12b-hexahydro-2-hydroxy-3-chloro-benz[d]indeno-[2,1-b]azepine

A. 1-(3-Methoxy-4-chlorophenyl)-1-indene

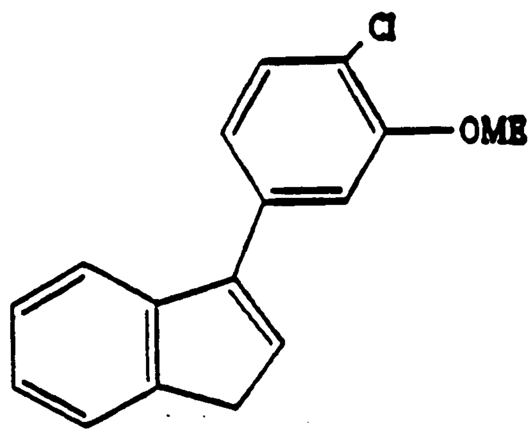

XXIII

Into a 500 mL three-neck flask fitted with a reflux condenser and addition funnel was placed 4.6 gm (.189 mmol) magnesium ribbon and one crystal of iodine. The flask was flushed with dry nitrogen while heating briefly with a flame. After cooling, 25 mL of a solution of 38.0 gm (0.172 mmol) 2-chloro-5-bromoanisole in 200 mL dry ether was added from the addition funnel, and the flask was warmed briefly to initiate reaction. Thereafter, the solution was added over the course of one hour at a rate which maintained a gentle reflux. When the addition was complete, the reaction was heated to reflux for an additional three

hours. The flask was cooled in an ice-salt bath to 0 degrees, and a solution of 1-indanone in 100 mL dry ether was added over 90 minutes while maintaining the reaction temperature at less than 10 degrees. Thereafter, the reaction was stirred at room temperature overnight, then quenched with 1M aqueous HCl until pH7. The aqueous layer was separated and extracted twice with 200 mL ether. The combined ether layers were dried ($MgSO_4$) and evaporated to an oil. This was purified by HPLC, eluting with 5% ethyl actetate in hexane to give 23.4 grams of desired product.

200 MHz NMR($CDCl_3$) d 3.51(d,2H,J=2Hz), 3.95(s,3H), 6.59(tr,1H,J=2Hz), 6.85-7.58(m,7H)

B. trans-1-(3-Methoxy-4-chlorophenyl)-2-indanamine

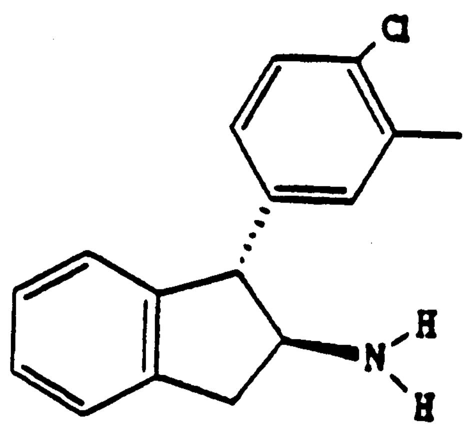

XXIV

A solution of 17.9 grams (69.7 mmol) of the compound from step A dissolved in 70 mL dry diglyme was treated with 11.6 mL of 2M borane-methyl sulfide complex in methylene chloride, and the mixture was stirred at 65° for 24 hours. A solution of 8.13 grams (71.8 mmol) hydroxylamine O-sulfonic acid in 50 mL dry diglyme was added, and stirring was continued at 100° for 6 hours. After cooling to room temperature, the reaction was quenched with 3N HCl until acidic, then stirred for two hours. The reaction was diluted with 300 mL water and extracted three times with 200 mL ethyl acetate. The aqueous layer was made basic with solid potassium hydroxide and extracted three times with 300 mL ethyl actetate. The organic layer was dried ($MgSO_4$) and evaporated. The residue was filtered through a silica gel column, eluting first with hexane and then with 10% methanol - methylene chloride to give 5.47 grams (28%) desired amine.

200 MHz NMR ($CDCl_3$) d 2.76(dd,1H,J=9,16 Hz), 3.24(dd,1H,J=8,16Hz),3.86 (d,1H,J=8Hz), 6.70-6.90-(m,3H),7.10-7.30(m,4H)

C. N-(2,2-diethoxyethyl)-trans-1-(3-Methoxy-4-chlorophenyl)-2-indanamine

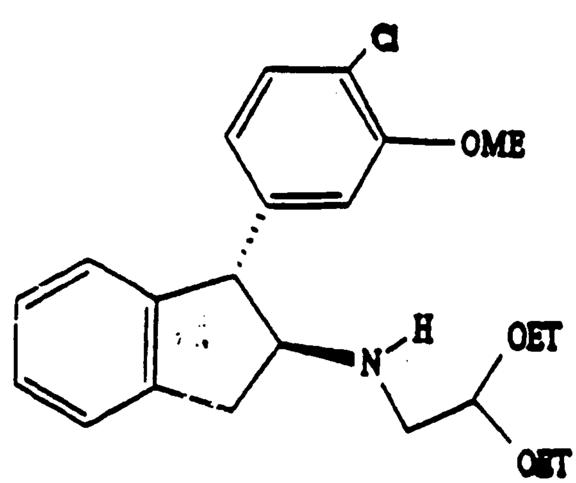

XXV

A mixture of 1.0 grams(3.65 mmol) of the amine from step B, 0.55 mL (3.65 mmol) 2-bromoacetaldehyde diethyl acetal, and 1.0 grams (7.3 mmol) anhydrous potassium carbonate in 35 mL dry DMF was stirred at 150° for 4 hours. After cooling to room temperature, the mixture was poured into 300 mL ether, washed with three 50 mL portions of water, dried ($MgSO_4$) and evaporated. The residue was purified by flash chromatography, eluting with 1:1 hexane-ethyl acetate to give 1.1 grams (77%) of the desired product as an oil.

200 MHz NMR($CDCl_3$) d 1.15(tr,6H,J=7Hz), 2.70-290(m,3H), 3.20-3.70(m,6H), 4.09(d,1H,J=8Hz), 4.55-(tr,1H,J=5Hz), 6.70-6.90(m,3H), 7.10-7.30(m,4H)

D. trans-7,7a,8,12b-tetrahydro-2-methoxy-3-chlorobenz[d]indeno-[2,1-b]azepine

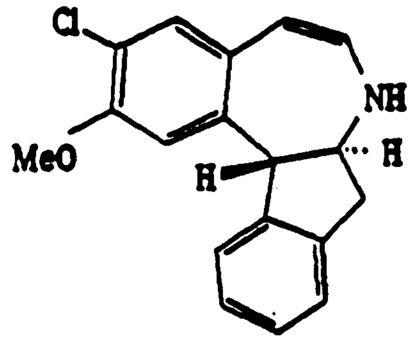

XXVI

A solution of the acetal from step C dissolved in 100 mL methylene chloride at 0° was treated with 100 mL trifluoromethanesulfonic acid added in a slow steady stream over 10 minutes. The mixture was stirred for five hours while coming to room temperature. The mixture was again cooled to 0° and cautiously quenched with saturated aqueous sodium bicarbonate until pH7. The mixture was extracted with three 100 mL portions of methylene chloride. The organic layer was dried ($MgSO_4$) and evaporated to give 0.57 grams of the enamine as a dark oil.

200 MHz NMR ($CDCl_3$) d 2.79(dd,1H,J=8Hz,15Hz), 3.50-3.59(m,2H), 3.78(s,3H), 4.27(d,1H,J=6Hz), 5.21(d,1H,J=10Hz), 6.23(dd,1H,J=5,10Hz), 6.99(s,1H), 7.18(s,1H), 7.20-7.55(m,4H)

E. trans-5,6,7,7a,8,12b-hexahydro-2-methoxy-3-chlorobenz[d]indeno-[2,1-b]azepine

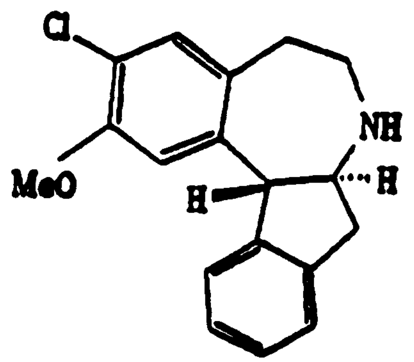

XXVII

The crude enamine from step D was dissolved in 20 mL absolute ethanol and treated with 0.12 grams (1.91 mmol) sodium cyanoborohydride. To this was added 0.108 mL glacial acetic acid, and the mixture was stirred at room temperature for 3 hours. The reaction was quenched with 10 mL 1M HCl and stirred for 30 minutes. The reaction was made basic with 30% NaOH and extracted into 250 mL ethyl acetate. The organic layer was dried ($MgSO_4$) and evaporated to give 350 mg (53%) of the product as an oil.

200 MHz NMR ($CDCl_3$) d 3.60-3.85(m,3H), 3.10-3.40(m,4H), 3.71(s,3H), 4.54(d,1H,J=8Hz), 7.07(s,1H), 7.16(s,1H), 7.20-7.40(m,4H)

F. trans-6-Methyl-5,6,7,7a,8,12b-hexahydro-2-methoxv-3-chloro-benz[d]indeno-[2,1-b]azepine

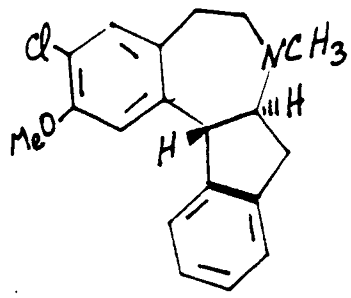

XXVIII

A solution of 300 mg (1.00 mmol) of the compound from step E was dissolved in 20 mL acetonitrile and 0.40mL (5.3 mmol) 37% aqueous formaldehyde was added followed by 0.10 grams (1.59 mmol) sodium cyanoborohydride. After 30 minutes, the solution was brought to pH 7 by dropwise addition of glacial acetic acid then stirred an additional 1 hour and 45 minutes. The solvent was removed under reduced pressure, and the residue was taken up into 125 mL ethyl acetate. This was washed with 50 mL 10% sodium bicarbonate, dried ($MgSO_4$) and evaporated to give an oil, which was purified by flash chromatography over silica gel, eluting with ethyl acetate to give 150 mg product as an oil.

200 MHz NMR ($CDCl_3$) d 2.17(dd,1H,J=12,12Hz), 2.69(m,2H) 2.37(s,3H), 2.90-3.40(m,4H), 3.77(s,3H), 4.67(d,1H,J=9Hz), 7.03(s,1H), 7.17(s,1H), 7.20-7.35(m,4H)

G. trans-6-Methyl-5,6,7,7a,8,12b-hexahydro-2-hydroxy-3-chloro-benz[d]indeno-[2,1-b]azepine Hydroxyamide

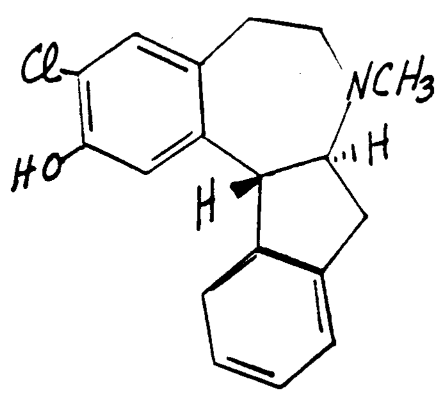

XXIX

A solution of 147 mg (0.47 mmol) of the product from Step F in 10 mL methylene chloride at -78° was treated with 90 µL (microliters) boron tribromide, and the mixture was stirred for 4 hours while coming to room temperature. The reaction was quenched with 10 mL dry methanol and stirred for 10 minutes. The solvent was evaporated under reduced pressure, and the residue was treated with a second 10 mL portion of methanol. After 10 minutes, the solvent was evaporated at 10 mm Hg and 50° for 45 minutes to give 180 mg (100%) of the crude hydrobromide.

200 MHz NMR(d6-DMSO) d 2.97(s,3H), 2.90-3.90(m,7H),5.02(d,1H,J=8Hz),6.99(s,1H),7.36(m,5H)

A portion was recrystallized from methanolether

| | | | |
|---|---|---|---|
| Calculated | C 56.79 | H 5.03 | N 3.68 |
| Found | C 56.40 | H 4.92 | N 3.55 |

By application of the above-described techniques and related procedures known to those skilled in the art, the compounds listed in Table II below may also be synthesized. For the combination of substituents shown in Table II, W, $R^{11}$ and $R^{12}$ are all hydrogen, (t) may be either benzene or thiophene fused in either the 2,3 or 3,2 position.

TABLE II

| X | Y | Z | $R^1$ | R |
|---|---|---|---|---|
| $OCH_3$ | OH | H | H | $CH_3$ |
| Cl | OH | H | H | $CH_3$ |
| $OCH_3$ | OH | H | H | $CH_3$ |
| H | OH | H | H | $CH_3$ |
| $CH_3$ | OH | H | H | $CH_3$ |
| Cl | OH | H | H | H |
| H | $NH_2$ | H | H | $CH_3$ |
| $CH_3$ | $NH_2$ | H | H | $CH_3$ |
| Cl | $NH_2$ | H | H | $CH_3$ |
| Cl | * | H | H | $CH_3$ |
| $CH_3O$ | * | H | H | $CH_3$ |

Note: * = $-OCON(CH_3)_2$

## Claims

1. A compound having the trans isomer structural formula I, and pharmaceutically acceptable salts thereof,

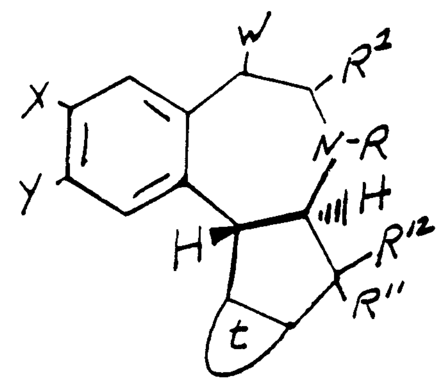

I

wherein:

R is hydrogen, alkyl, $-CH_2CH=CH_2$ or $-CH_2-\triangleleft$ ;

$R^1$, $R^{11}$ and $R^{12}$ are the same or different and each is hydrogen or alkyl;

X is hydrogen, halo, alkyl, alkylthio, alkylsulfinyl, alkylsulfonyl, hydroxy, alkoxy or trifluoromethyl;

Y is hydrogen, hydroxy, alkoxy,

$$-O\overset{O}{\overset{\|}{C}}NR^2R^3,\quad -O\overset{O}{\overset{\|}{C}}-R^9,\quad -NR^1_2\ ,\quad -NH\overset{O}{\overset{\|}{C}}R^1 \text{ or } -O\overset{O}{\overset{\|}{P}}(OH)OR^1;$$

where $R^1$ is as defined above;

W is hydrogen, hydroxy or alkoxy;

ring (t) represents a fused thiophene or fused benzene ring said fused benzene ring optionally being substituted with a substitutent Z as defined below;

$R^2$ and $R^3$ are independently hydrogen (provided that both are not hydrogen), alkyl, aralkyl, cycloalkyl, aryl, hydroxyalkyl, or alkoxyalkyl;

in addition, when one of $R^2$ and $R^3$ is as defined above, the other may be $-R^4NR^5R^6$ {wherein $R^4$ is alkanediyl, $R^5$ is hydrogen or alkyl and $R^6$ is alkyl, or $R^5$ and $R^6$ together with the nitrogen atom form a 1-azetidinyl, 1-pyrrolidinyl, 1-piperidinyl, 1-(4-alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-alkylpiperazinyl), 1-(4-alkoxyalkylpiperazinyl), 1-(4-hydroxyalkylpiperazinyl), 1-(3-hydroxyazetidinyl), 1-(3-alkoxyazetidinyl), 1-(3-hydroxypyrrolidinyl), 1-(3-alkoxypyrrolidinyl), 1-(3- or 4-hydroxypiperidinyl), 1-(3- or 4-alkoxypiperidinyl), 1-(4-oxopiperidinyl) or 1-(3-oxopyrrolidinyl) ring;

in still further addition, when $R^2$ is hydrogen, $R^3$ may be $-CHR^7CO_2R^8$, wherein $R^7$ and $R^8$ are independently hydrogen, alkyl or aralkyl;

$R^9$ is alkyl, aralkyl, aryl, alkoxyalkyl, aryloxyalkyl, aralkoxyalkyl, cycloalkylalkyl, alkoxycarbonylalkyl, cycloalkyl, 1-adamantyl, cycloalkoxyalkyl, alkoxy, aralkoxy, cycloalkoxy, aryloxy or $-CHR^7NHR^8$ {wherein $R^7$ and $R^8$ are as defined above};

Z is X as defined above, amino, alkylamino or

$$-NH\overset{O}{\overset{\|}{C}}R^{10}$$

{wherein $R^{10}$ is hydrogen, alkyl or aryl}; and

alkyl and the alkyl portions of alkoxy and aralkyl represent straight or branched carbon chains having 1 to 6 carbon atoms, and aryl and the aryl portions of aralkyl represent unsubstituted phenyl and phenyl monosubstituted by alkyl, hydroxy, alkoxy, halo or trifluoromethyl.

2. A compound wherein the substituents are as defined in claim 1 with the proviso that when X is $OCH_3$, Y is OH, Z and $R^1$ are both H, R cannot be $CH_3$.

3. A compound as defined in claim 1 or claim 2 wherein W, $R^{11}$ and $R^{12}$ are all hydrogen.

4. A compound as defined in any of claims 1-3 above
wherein Y is hydroxy;

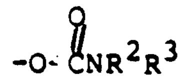

where $R^2$ and $R^3$ are both alkyl or one of $R^2$ and $R^3$ is hydrogen and the other is alkyl; $-NHR^1$ where $R^1$ is hydrogen or methyl;

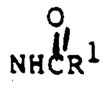

wherein $R^1$ is hydrogen or methyl; or

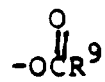

wherein $R^9$ is as defined in claim 1;
X is hydrogen, alkyl, halogen or alkoxy;
Z is hydrogen, halogen, alkyl, hydroxy or alkoxy;
R is methyl; and,
$R^1$ is hydrogen or methyl.

5. A compound as defined in any of claims 1-4 above wherein
Y is hydroxy, amino,

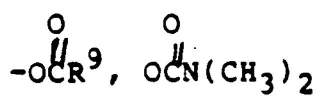

or $-NHCH_3$ wherein $R^9$ is as defined in claim 1;
ring (t) is a fused benzene ring;
Z is hydrogen, halo, alkyl or $-OR^1$ where $R^1$ is hydrogen or alkyl;
X is hydrogen, methyl, methoxy, chloro or bromo;
R is methyl; and
$R^1$ is hydrogen;

6. A compound as defined in claim 1 which is:
(1) trans 5,6,7,7a,8,12b hexahydro-2-hydroxy-3-chloro-7-methyl-benz[d]indeno-[2,1-b]azepine or a pharmaceutically acceptable salt thereof;
(2) (±)-trans 5,6,7,7a,8,12b-hexahydro-2-hydroxy-3-chloro-7-methyl-benz[d]-indeno[2,1-b]azepine or a pharmaceutically acceptable salt thereof;
(3) (-)-trans 5,5,7,7a,8,12b-hexahydro-2-hydroxy-3-chloro-7-methyl-benz[d]-indeno[2,1-b]azepine or a pharmaceutically acceptable salt thereof;
(4) trans-5,6,7,7a,8,12b-hexahydro-2-hydroxy-3-methoxy-7-methyl-benz[d]-indeno[2,1-b]azepine or a pharmaceutically acceptable salt thereof;
(5) trans - 5,6,7,7a,8,12b-hexahydro-2-amino-3-chloro-7-methyl-benz[d]-indeno[2,1-b]azepine or a pharmaceutically acceptable salt thereof;

(6) trans - 5,6,7,7a,8,12b-hexahydro-2-hydroxy-7-methyl-benz[d]-indeno[2,1-b]azepine or a pharmaceutically acceptable salt thereof;

(7) trans - 5,6,7,7a,8,12b-hexahydro-3,7-dimethyl, 2-hydroxy-benz[d]-indeno[2,1-b]azepine or a pharmaceutically acceptable salt thereof;

(8) trans - 5,6,7,7a,8,12b-hexahydro-3-chloro-7-cyclo-propylmethyl-2-hydroxy-benz[d]indeno[2,1-b]-azepine, or a pharmaceutically acceptable salt thereof;

(9) trans - 5,6,7,7a,8,12b-hexahydro-7-allyl-3-chloro-2-hydroxy-benz[d]-indeno[2,1-b]azepine, or a pharmaceutically acceptable salt thereof;

(10) trans - 5,6,7,7a,8,12b-hexahydro-3-chloro-2-hydroxy-7,8,8-trimethyl-benz[d]-indeno[2,1-b]-azepine, or a pharmaceutically acceptable salt thereof; and

(11) trans - 3-chloro-5,6,7,7a,8,11b-hexahydro-7-methyl-thieno[2',3':4,5]cyclopenta[1,2-a] [3]-benzazepine-2-ol, or a pharmaceutically acceptable salt thereof.

**7.** The compound of claim 1 which is:

(1) trans - 5,6,7,7a,8,12b-hexahydro-2-hydroxy-3-chloro-7-methyl-benz[d]indeno-[2,1-b]azepine or a pharmaceutically acceptable salt thereof;

(2) (±)-trans-5,6,7,7a,8,12b-hexahydro-2-hydroxy-3-chloro-7-methyl-benz[d]-indeno[2,1-b]azepine, or a pharmaceutically acceptable salt thereof;

(3) (-)-trans-5,6,7,7a,8,12b-hexahydro-2-hydroxy-3-chloro-7-methyl-benz[d]-indeno[2,1-b]azepine or a pharmaceutically acceptable salt thereof;

(4) trans-5,6,7,7a,8,12b-hexahydro-2-hydroxy-3-methoxy-7-methyl-benz[d]-indeno[2,1-b]azepine, or a pharmaceutically acceptable salt thereof;

(5) trans - 5,6,7,7a,8,12b-hexahydro-2-amino-3-chloro-7-methyl-benz[d]-indeno[2,1-b]azepine, or a pharmaceutically acceptable salt thereof;

(6) trans - 5,6,7,7a,8,12b-hexahydro-2-hydroxy-7-methyl-benz[d]-indeno[2,1-b]azepine, or a pharmaceutically acceptable salt thereof;

(7) trans - 5,6,7,7a,8,12b-hexahydro-3,7-dimethyl, 2-hydroxy-benz[d]-indeno[2,1-b]azepine, or a pharmaceutically acceptable salt thereof; and

(8) trans - 5,6,7,7a,8,12b-hexahydro-2-hydroxy-3-chloro-7,8,8-tri-methyl-benz[d]indeno[2,1-b]azepine.

**8.** A pharmaceutical composition which comprises a compound as defined in any one of claims 1-7 in combination with a pharmaceutically acceptable carrier.

**9.** The use of a compound of any one of claims 1-6 for the preparation of a pharmaceutical composition useful in the treatment of psychoses, in the treatment of pain, in the treatment of depression and/or as a renal vasodilator.

**10.** A compound of the formula II, XI, XIII or XIV

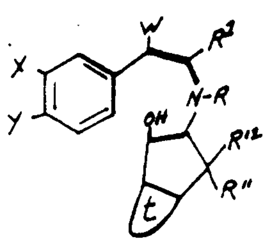

II

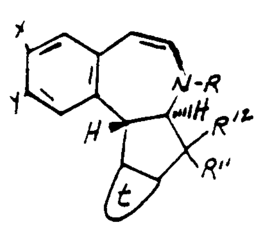

XI

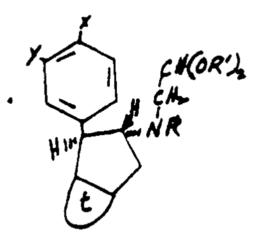

XIII

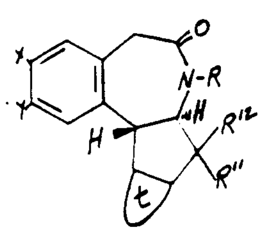

XIV

wherein

R is hydrogen, alkyl, $-CH_2CH=CH_2$ or $-CH_2-\triangleleft$ ;

$R^1$, $R^{11}$ and $R^{12}$ are the same or different and each is hydrogen or alkyl;

X is hydrogen, halo, alkyl, alkylthio, alkylsulfinyl, alkylsulfonyl, hydroxy, alkoxy or trifluoromethyl;

Y is hydrogen, hydroxy, alkoxy,

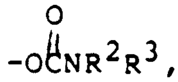, 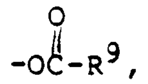, $-NR^{1}_{2}$, 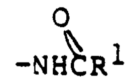 or 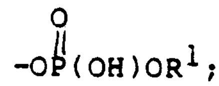;

W is hydrogen, hydroxy or alkoxy;

ring (t) represents a fused thiophene or fused benzene ring, said fused benzene ring optionally being substituted with a substituent Z as defined below;

$R^2$ and $R^3$ are independently hydrogen (provided that both are not hydrogen), alkyl, aralkyl, cycloalkyl, aryl, hydroxyalkyl, or alkoxyalkyl;

in addition, when one of $R^2$ and $R^3$ is as defined above, the other may be $-R^4NR^5R^6$ {wherein $R^4$ is alkylene, $R^5$ is a hydrogen or alkyl and $R^6$ is alkyl, or $R^5$ and $R^6$ together with the nitrogen atom form a 1-azetidinyl, 1-pyrrolidinyl, 1-piperidinyl, 1-(4-alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-alkylpiperazinyl), 1-(4-alkoxyalkylpiperazinyl), 1-(4-hydroxyalkylpiperazinyl), 1-(3-hydroxyazetidinyl), 1-(3-alkoxyazetidinyl), 1-(3-hydroxypyrrolidinyl), 1-(3-alkoxypyrrolidinyl), 1-(3- or 4-hydroxypiperidinyl), 1-(3- or 4-alkoxypiperidinyl), 1-(4-oxopiperidinyl) or 1-(4-oxopyrrolidinyl) ring;

in still further addition, when $R^2$ is hydrogen, $R^3$ may additionally be $-CHR^7CO_2R^8$, wherein $R^7$ and $R^8$ are independently hydrogen, alkyl or aralkyl;

$R^9$ is alkyl, aralkyl, aryl, alkoxyalkyl, aryloxyalkyl, aralkoxyalkyl, cycloalkylalkyl, alkoxycarbonylalkyl, cycloalkyl, 1-adamantyl, cycloalkoxyalkyl, alkoxy, aralkoxy, cycloalkoxy, aryloxy or $-CHR^7NHR^8$ {wherein $R^7$ and $R^8$ are as defined above};

R' is a $C_1$-$C_3$ alkyl;

Z is X as defined above, nitro, amino, alkylamino and

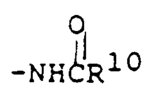

{wherein $R^{10}$ is hydrogen, alkyl or aryl}; and

alkyl and the alkyl portions of alkoxy and aralkyl represent straight or branched carbon chains having 1 to 6 carbon atoms, and aryl and the aryl portions of aralkyl represent unsubstituted phenyl and phenyl mono-substituted by alkyl, hydroxy, alkoxy, halo or trifluoromethyl.

**11.** A process for producing a compound having the structural formula I

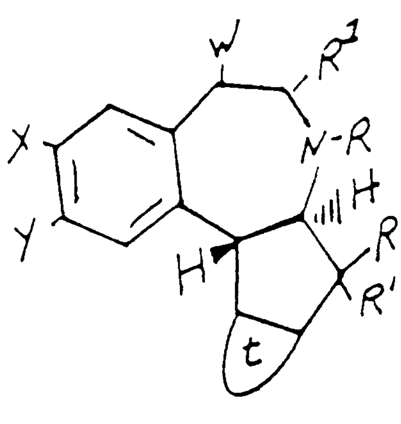

I

wherein:

R is hydrogen, alkyl, $-CH_2CH=CH_2$ or 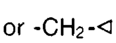 ;

$R^1$, $R^{11}$ and $R^{12}$ are the same or different and each is hydrogen or alkyl;

X is hydrogen, halo, alkyl, alkylthio, alkylsulfinyl, alkylsulfonyl, hydroxy, alkoxy or trifluoromethyl;

Y is hydrogen, hydroxy, alkoxy,

$$-O\overset{O}{\overset{\|}{C}}NR^2R^3,\ -O\overset{O}{\overset{\|}{C}}-R^9,\ -NR^1_2\ ,\ -NH\overset{O}{\overset{\|}{C}}R^1 \text{ or } -O\overset{O}{\overset{\|}{P}}(OH)OR^1;$$

W is hydrogen, hydroxy or alkoxy;

ring (t) represents a fused thiophene or fused benzene ring said fused benzene ring optionally being substituted with a substitutent Z as defined below;

$R^2$ and $R^3$ are independently hydrogen (provided that both are not hydrogen), alkyl, aralkyl, cycloalkyl, aryl, hydroxyalkyl, or alkoxyalkyl;

in addition, when one of $R^2$ and $R^3$ is as defined above, the other may be $-R^4NR^5R^6$ {wherein $R^4$ is alkanediyl, $R^5$ is hydrogen or alkyl and $R^6$ is alkyl, or $R^5$ and $R^6$ together with the nitrogen atom form a 1-azetidinyl, 1-pyrrolidinyl, 1-piperidinyl, 1-(4-alkylpiperazinyl), 4-morpholinyl or 1-hexahydroazepinyl group},

in further addition, $R^2$ and $R^3$ together with the nitrogen atom may form a 1-azetidinyl, 1-pyrrolidinyl, 1-piperidinyl, 4-morpholinyl, 1-(4-alkylpiperazinyl), 1-(4-alkoxyalkylpiperazinyl), 1-(4-hydroxyalkylpiperazinyl), 1-(3-hydroxyazetidinyl), 1-(3-alkoxyazetidinyl), 1-(3-hydroxypyrrolidinyl), 1-(3-alkoxypyrrolidinyl), 1-(3- or 4-hydroxypiperidinyl), 1-(3- or 4-alkoxypiperidinyl), 1-(4-oxopiperidinyl) or 1-(3-oxopyrrolidinyl) ring;

in still further addition, when $R^2$ is hydrogen, $R^3$ may be $-CHR^7CO_2R^8$, wherein $R^7$ and $R^8$ are independently hydrogen, alkyl or aralkyl;

$R^9$ is alkyl, aralkyl, aryl, alkoxyalkyl, aryloxyalkyl, aralkoxyalkyl, cycloalkylalkyl, alkoxycarbonylalkyl, cycloalkyl, 1-adamantyl, cycloalkoxyalkyl, alkoxy, aralkoxy, cycloalkoxy, aryloxy or $-CHR^7NHR^8$ {wherein $R^7$ and $R^8$ are as defined above};

R' is a $C_1$-$C_3$ alkyl;

Z is X as defined above, amino, alkylamino or

$$-NH\overset{O}{\overset{\|}{C}}R^{10}$$

{wherein $R^{10}$ is hydrogen, alkyl or aryl}; and

alkyl and the alkyl portions of alkoxy and aralkyl represent straight or branched carbon chains having 1 to 6 carbon atoms, and aryl and the aryl portions of aralkyl represent unsubstituted phenyl and phenyl mono-substituted by alkyl, hydroxy, alkoxy, halo or trifluoromethyl;

characterized by;

(a) intramolecular condensation of a compound of the formula

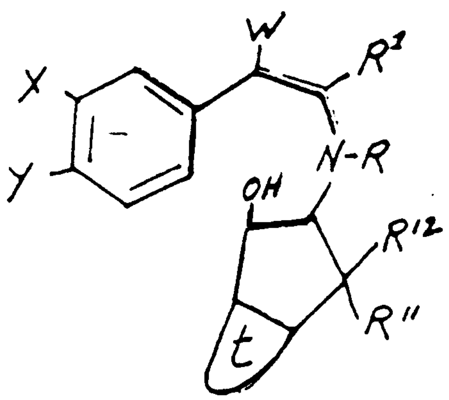

II

(b) by reacting a compound of the formula

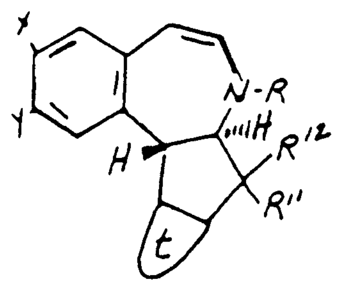

XI

with a reducing agent;

(c) contacting a compound of the formula

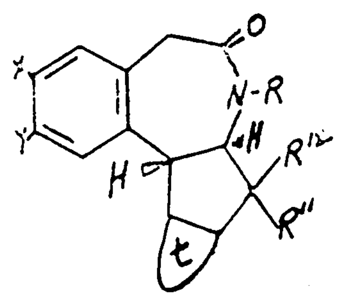

XIV

with a reducing agent; or reacting a compound of formula XIII

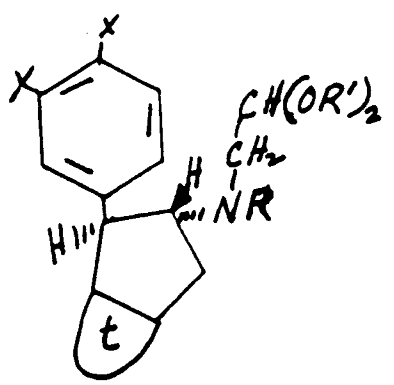

XIII

with a strong acid.

**12.** The process as defined in claim 11 wherein the substituents are as defined in claim 11 with the proviso that when X is $OCH_3$, Y is OH, Z and $R^1$ are both H, R cannot be $CH_3$.

**13.** A method for making a pharmaceutical composition comprising mixing a compound of formula I with a pharmaceutically acceptable carrier.

**Patentansprüche**

**1.** Verbindung mit der trans-Isomerstrukturformel I und der pharmazeutisch annehmbaren Salze derselben

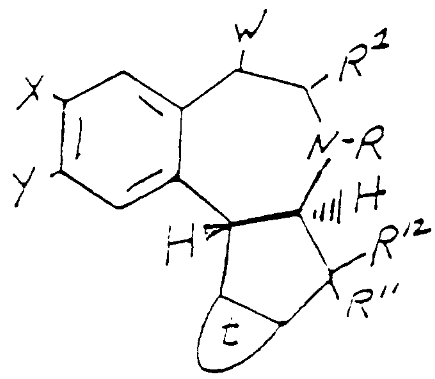

I

worin:

R — Wasserstoff, Alkyl, $-CH_2CH=CH_2$ oder $-CH_2-\lhd$ ist;

$R^1$, $R^{11}$ und $R^{12}$ — gleich oder verschieden sind und jeweils Wasserstoff oder Alkyl sind;

X — Wasserstoff, Halogen, Alkyl, Alkylthio, Akylsulfinyl, Alkylsulfonyl, Hydroxy, Alkoxy oder Trifluormethyl ist;

Y — Wasserstoff, Hydroxy, Alkoxy,

$$-O\overset{O}{\overset{\|}{C}}NR^2R^3,\ -O\overset{O}{\overset{\|}{C}}-R^9,\ -NR^1_2,\ -NH\overset{O}{\overset{\|}{C}}R^1 \text{ oder } -O\overset{O}{\overset{\|}{P}}(OH)OR^1$$

ist, worin $R^1$ wie vorstehend definiert ist;

W — Wasserstoff, Hydroxy oder Alkoxy ist;

Ring (t) einen kondensierten Thiophen-Ring oder kondensierten Benzol-Ring bezeichnet, wobei der kondensierte Benzol-Ring gegebenenfalls mit einem Substituenten Z substituiert ist, wie nachstehend definiert,

$R^2$ und $R^3$ — unabhängig voneinander Wasserstoff (vorausgesetzt, daß nicht beide Wasserstoff sind), Alkyl, Aralkyl, Cycloalkyl, Aryl, Hydroxyalkyl oder Alkoxyalkyl sind;
zusätzlich, wenn einer der Substituenten $R^2$ und $R^3$ wie vorstehend definiert ist, der andere $-R^4NR^5R^6$ sein kann {worin $R^4$ Alkandiyl ist, $R^5$ Wasserstoff oder Alkyl ist und $R^6$ Alkyl ist, oder $R^5$ und $R^6$ zusammen mit dem Stickstoff-Atom eine 1-Azetidinyl-, 1-Pyrrolidinyl-, 1-Piperidinyl-, 1-(4-Alkylpiperazinyl)-, 4-Morpholinyl- oder 1-Hexahydroazepinyl-Gruppe bilden},
weiterhin zusätzlich $R^2$ und $R^3$ zusammen mit dem Stickstoff-Atom einen 1-Azetidinyl-, 1-Pyrrolidinyl-, 1-Piperidinyl-, 4-Morpholinyl-, 1-(4-Alkylpiperazinyl)-, 1-(4-Alkoxyalkylpiperazinyl)-, 1-(4-Hydroxyalkylpiperazinyl), 1-(3-Hydroxyazetidinyl)-, 1-(3-Alkoxyazetidinyl)-, 1-(3-Hydroxypyrrolidinyl)-, 1-(3-Alkoxypyrrolidinyl)-, 1-(3- oder 4-Hydroxypiperidinyl)-, 1-(3- oder 4-Alkoxypiperidinyl)-, 1-(4-Oxopiperidinyl)- oder 1-(3-Oxopyrrolidinyl)-Ring bilden können;
noch weiterhin zusätzlich dann, wenn $R^2$ Wasserstoff ist, $R^3$ $-CHR^7CO_2R^8$ sein kann, worin $R^7$ und $R^8$ unabhängig voneinander Wasserstoff, Alkyl, oder Aralkyl sind,

$R^9$ — Alkyl, Aralkyl, Aryl, Alkoxyalkyl, Aryloxyalkyl, Aralkoxyalkyl, Cycloalkylalkyl, Alkoxycarbonylalkyl, Cycloalkyl, 1-Adamantyl, Cycloalkoxyalkyl, Alkoxy, Aralkoxy, Cycloalkoxy, Aryloxy oder $-CHR^7NHR^8$ sein kann (worin $R^7$ und $R^8$ wie vorstehend definiert sind);

Z — X ist, wie vorstehend definiert, Amino, Alkylamino oder

$$-NH\overset{O}{\overset{\|}{C}}R^{10}$$

ist

(worin $R^{10}$ Wasserstoff, Alkyl oder Aryl ist); und

Alkyl und die Alkyl-Teile von Alkoxy und Aralkyl lineare oder verzweigte Kohlenstoff-Ketten mit 1 bis 6 Kohlenstoff-Atomen bezeichnen, und Aryl und die Aryl-Teile von Aralkyl unsubstituiertes Phenyl bezeichnen, sowie Phenyl, das monosubstituiert ist durch Alkyl, Hydroxy, Alkoxy, Halogen oder Trifluormethyl.

2. Verbindung, worin die Substituenten wie in Anspruch 1 definiert sind, mit der Maßgabe, daß wenn X $OCH_3$ ist, Y OH ist, Z und $R^1$ beide H sind, R nicht $CH_3$ sein kann.

3. Verbindung nach Anspruch 1 oder Anspruch 2, worin W, $R^{11}$ und $R^{12}$ sämtlich Wasserstoff sind.

4. Verbindung nach irgendeinem der Ansprüche 1 bis 3, worin
   Y Hydroxy,

$$-O-\overset{\overset{O}{\|}}{C}NR^2R^3,$$

worin $R^2$ und $R^3$ beide Alkyl sind oder eines der $R^2$ und $R^3$ Wasserstoff ist und das andere Alkyl ist, $-NHR^1$, worin $R^1$ Wasserstoff oder Methyl ist,

$$NH\overset{\overset{O}{\|}}{C}R^1,$$

worin $R^1$ Wasserstoff oder Methyl ist, oder

$$-O\overset{\overset{O}{\|}}{C}R^9$$

ist, worin $R^9$ wie in Anspruch 1 definiert ist;
   X Wasserstoff, Alkyl, Halogen oder Alkoxy ist;
   Z Wasserstoff, Halogen, Alkyl, Hydroxy oder Alkoxy ist;
   R Methyl ist; und
   $R^1$ Wasserstoff oder Methyl ist.

5. Verbindung nach nach irgendeinem der Ansprüche 1 bis 4, worin
   Y Hydroxy, Amino,

$$-O\overset{\overset{O}{\|}}{C}R^9, \quad O\overset{\overset{O}{\|}}{C}N(CH_3)_2$$

oder $-NHCH_3$ ist, worin $R^9$ wie in Anspruch 1 definiert ist;
   der Ring (t) ein kondensierter Benzol-Ring ist;
   Z Wasserstoff, Halogen, Alkyl oder $-OR^1$ ist, worin $R^1$ Wasserstoff oder Alkyl ist;
   X Wasserstoff, Methyl, Methoxy, Chlor oder Brom ist;
   R Methyl ist;
   $R^1$ Wasserstoff ist.

6. Verbindung nach Anspruch 1, die
   (1) trans-5,6,7,7a,8,12b-Hexahydro-2-hydroxy-3-chlor-7-methyl-benz[d]indeno[2,1-b]azepin oder ein

pharmazeutisch annehmbares Salz desselben,
(2) (±)-trans-5,6,7,7a,8,12b-Hexahydro-2-hydroxy-3-chlor-7-methyl-benz[d]indeno[2,1-b]azepin oder ein pharmazeutisch annehmbares Salz desselben,
(3) (-)-trans-5,6,7,7a,8,12b-Hexahydro-2-hydroxy-3-chlor-7-methyl-benz[d]indeno[2,1-b]azepin oder ein pharmazeutisch annehmbares Salz desselben,
(4) trans-5,6,7,7a,8,12b-Hexahydro-2-hydroxy-3-methoxy-7-methyl-benz[d]indeno[2,1-b]azepin oder ein pharmazeutisch annehmbares Salz desselben,
(5) trans-5,6,7,7a,8,12b-Hexahydro-2-amino-3-chlor-7-methyl-benz[d]indeno[2,1-b]azepin oder ein pharmazeutisch annehmbares Salz desselben,
(6) trans-5,6,7,7a,8,12b-Hexahydro-2-hydroxy-7-methyl-benz[d]indeno[2,1-b]azepin oder ein pharmazeutisch annehmbares Salz desselben,
(7) trans-5,6,7,7a,8,12b-Hexahydro-3,7-dimethyl-2-hydroxy-benz[d]indeno[2,1-b]azepin oder ein pharmazeutisch annehmbares Salz desselben,
(8) trans-5,6,7,7a,8,12b-Hexahydro-3-chlor-7-cyclopropylmethyl-2-hydroxy-benz[d]indeno[2,1-b]-azepin oder ein pharmazeutisch annehmbares Salz desselben,
(9) trans-5,6,7,7a,8,12b-Hexahydro-7-allyl-3-chlor-2-hydroxy-benz[d]indeno[2,1-b]azepin oder ein pharmazeutisch annehmbares Salz desselben,
(10) trans-5,6,7,7a,8,12b-Hexahydro-3-chlor-2-hydroxy7,8,8-trimethyl-benz[d]indeno[2,1-b]azepin oder ein pharmazeutisch annehmbares Salz desselben, und
(11) trans-3-Chlor-5,6,7,7a,8,11b-hexahydro-7-methyl-thieno[2',3':4,5]cyclopenta[1,2-a][3]benzazepin-2-ol oder ein pharmazeutisch annehmbares Salz desselben ist.

**7.** Verbindung nach Anspruch 1, die
(1) trans-5,6,7,7a,8,12b-Hexahydro-2-hydroxy-3-chlor-7-methyl-benz[d]indeno[2,1-b]azepin oder ein pharmazeutisch annehmbares Salz desselben,
(2) (±)-trans-5,6,7,7a,8,12b-Hexahydro-2-hydroxy-3-chlor-7-methyl-benz[d]indeno[2,1-b)azepin oder ein pharmazeutisch annehmbares Salz desselben,
(3) (-)-trans-5,6,7,7a,8,12b-Hexahydro-2-hydroxy-3-chlor-7-methyl-benz[d]indeno[2,1-b]azepin oder ein pharmazeutisch annehmbares Salz desselben,
(4) trans-5,6,7,7a,8,12b-Hexahydro-2-hydroxy-3-methoxy-7-methyl-benz[d]indeno[2,1-b]azepin oder ein pharmazeutisch annehmbares Salz desselben,
(5) trans-5,6,7,7a,8,12b-Hexahydro-2-amino-3-chlor-7-methyl-benz[d]indeno[2,1-b]azepin oder ein pharmazeutisch annehmbares Salz desselben,
(6) trans-5,6,7,7a,8,12b-Hexahydro-2-hydroxy-7-methyl-benz[d]indeno[2,1-b]azepin oder ein pharmazeutisch annehmbares Salz desselben,
(7) trans-5,6,7,7a,8,12b-Hexahydro-3,7-dimethyl-2-hydroxy-benz[d]indeno[2,1-b]azepin oder ein pharmazeutisch annehmbares Salz desselben, und
(8) trans-5,6,7,7a,8,12b-Hexahydro-2-hydroxy-3-chlor-7,8,8-trimethyl-benz[d]indeno[2,1-b]azepin ist.

**8.** Pharmazeutische Zusammensetzung, umfassend eine Verbindung nach irgendeinem der Ansprüche 1 bis 7 in Kombination mit einem pharmazeutisch annehmbaren Träger.

**9.** Verwendung einer Verbindung nach irgendeinem der Ansprüche 1 bis 6 zur Herstellung einer pharmazeutischen Zusammensetzung, die bei der Behandlung von Psychosen, bei der Behandlung von Schmerzen, bei der Behandlung von Depressionen und/oder als renales gefäßerweiterndes Mittel brauchbar ist.

**10.** Verbindung der Formel II, XI, XIII oder XIV

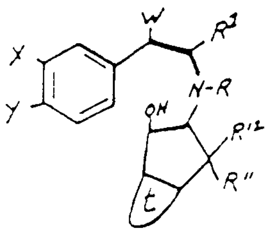

XI

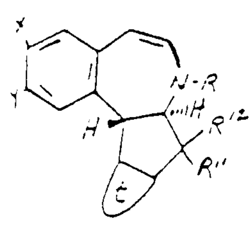

V

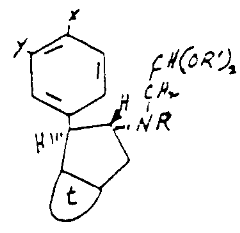

XIII

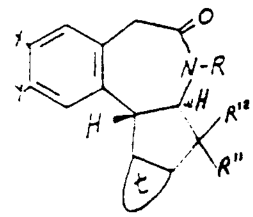

XII

worin:

R Wasserstoff, Alkyl, $-CH_2CH=CH_2$ oder $-CH_2-\triangleleft$ ist;

$R^1$, $R^{11}$ und $R^{12}$ gleich oder verschieden sind und jeweils Wasserstoff oder Alkyl sind;

X Wasserstoff, Halogen, Alkyl, Alkylthio, Akylsulfinyl, Alkylsulfonyl, Hydroxy, Alkoxy oder Trifluormethyl ist;

Y Wasserstoff, Hydroxy, Alkoxy,

$$-O\overset{O}{\overset{\|}{C}}NR^2R^3,\quad -O\overset{O}{\overset{\|}{C}}-R^9,\quad -NR^1_2,\quad -NH\overset{O}{\overset{\|}{C}}R^1 \text{ oder } -O\overset{O}{\overset{\|}{P}}(OH)OR^1$$

ist;

W Wasserstoff, Hydroxy oder Alkoxy ist;

Ring (t) einen kondensierten Thiophen-Ring oder kondensierten Benzol-Ring bezeichnet, wobei der kondensierte Benzol-Ring gegebenenfalls mit einem Substituenten Z substituiert ist, wie nachstehend definiert,

$R^2$ und $R^3$ unabhängig voneinander Wasserstoff (vorausgesetzt, daß nicht beide Wasserstoff sind), Alkyl, Aralkyl, Cycloalkyl, Aryl, Hydroxyalkyl oder Alkoxyalkyl sind;
zusätzlich, wenn einer der Substituenten $R^2$ und $R^3$ wie vorstehend definiert ist, der andere $-R^4NR^5R^6$ sein kann {worin $R^4$ Alkylen ist, $R^5$ Wasserstoff oder Alkyl ist und $R^6$ Alkyl ist, oder $R^5$ und $R^6$ zusammen mit dem Stickstoff-Atom eine 1-Azetidinyl-, 1-Pyrrolidinyl-, 1-Piperidinyl-, 1-(4-Alkylpiperazinyl)-, 4-Morpholinyl- oder 1-Hexahydroazepinyl-Gruppe bilden},
weiterhin zusätzlich $R^2$ und $R^3$ zusammen mit dem Stickstoff-Atom einen 1-Azetidinyl-, 1-Pyrrolidinyl-, 1-Piperidinyl-, 4-Morpholinyl-, 1-(4-Alkylpiperazinyl)-, 1-(4-Alkoxyalkylpiperazinyl)-, 1-(4-Hydroxyalkylpiperazinyl), 1-(3-Hydroxyazetidinyl)-, 1-(3-Alkoxyazetidinyl)-, 1-(3-Hydroxypyrrolidinyl)-, 1-(3- oder 4-Alkoxypiperidinyl)-, 1-(4-Oxopiperidinyl)- oder 1-(4-Oxopyrrolidinyl)-Ring bilden können;
noch weiterhin zusätzlich, wenn $R^2$ Wasserstoff ist, $R^3$ zusätzlich $-CHR^7CO_2R^8$ sein kann, worin $R^7$ und $R^8$ unabhängig voneinander Wasserstoff, Alkyl, oder Aralkyl sind,

$R^9$ Alkyl, Aralkyl, Aryl, Alkoxyalkyl, Aryloxyalkyl, Aralkoxyalkyl, Cycloalkylalkyl, Alkoxycarbonylalkyl, Cycloalkyl, 1-Adamantyl, Cycloalkoxyalkyl, Alkoxy, Aralkoxy, Cycloalkoxy, Aryloxy oder $-CHR^7NHR^8$ sein kann (worin $R^7$ und $R^8$ wie vorstehend definiert sind);

R' ein $C_1$-$C_3$-Alkyl ist;

Z X ist, wie vorstehend definiert, Nitro, Amino, Alkylamino und

$$-NH\overset{O}{\overset{\|}{C}}R^{10}$$

ist
(worin $R^{10}$ Wasserstoff, Alkyl oder Aryl ist); und

Alkyl und die Alkyl-Teile von Alkoxy und Aralkyl lineare oder verzweigte Kohlenstoff-Ketten mit 1 bis 6 Kohlenstoff-Atomen bezeichnen, und Aryl und die Aryl-Teile von Aralkyl unsubstituiertes Phenyl bezeichnen, sowie Phenyl, das monosubstituiert ist durch Alkyl, Hydroxy, Alkoxy, Halogen oder

Trifluormethyl.

**11.** Verfahren zur Herstellung einer Verbindung mit der Strukturformel I

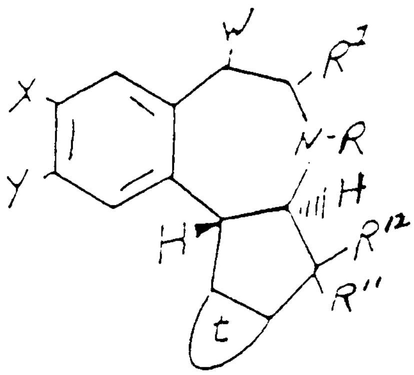

I

worin:

| | |
|---|---|
| R | Wasserstoff, Alkyl, $-CH_2CH=CH_2$ oder $-CH_2-$◁ ist; |
| $R^1$, $R^{11}$ und $R^{12}$ | gleich oder verschieden sind und jeweils Wasserstoff oder Alkyl sind; |
| X | Wasserstoff, Halogen, Alkyl, Alkylthio, Akylsulfinyl, Alkylsulfonyl, Hydroxy, Alkoxy oder Trifluormethyl ist; |
| Y | Wasserstoff, Hydroxy, Alkoxy, |

$$-O\overset{O}{\overset{\|}{C}}NR^2R^3,\ -O\overset{O}{\overset{\|}{C}}-R^9,\ -NR^1_2,\ -NH\overset{O}{\overset{\|}{C}}R^1 \text{ oder } -O\overset{O}{\overset{\|}{P}}(OH)OR^1$$

ist;

| | |
|---|---|
| W | Wasserstoff, Hydroxy oder Alkoxy ist; |

Ring (t) einen kondensierten Thiophen-Ring oder kondensierten Benzol-Ring bezeichnet, wobei der kondensierte Benzol-Ring gegebenenfalls mit einem Substituenten Z substituiert ist, wie nachstehend definiert;

| | |
|---|---|
| $R^2$ und $R^3$ | unabhängig voneinander Wasserstoff (vorausgesetzt, daß nicht beide Wasserstoff sind), Alkyl, Aralkyl, Cycloalkyl, Aryl, Hydroxyalkyl oder Alkoxyalkyl sind; zusätzlich, wenn einer der Substituenten $R^2$ und $R^3$ wie vorstehend definiert ist, der andere $-R^4NR^5R^6$ sein kann {worin $R^4$ Alkandiyl ist, $R^5$ Wasserstoff oder Alkyl ist und $R^6$ Alkyl ist, oder $R^5$ und $R^6$ zusammen mit dem Stickstoff-Atom eine 1-Azetidinyl-, 1-Pyrrolidinyl-, 1-Piperidinyl-, 1-(4-Alkylpiperazinyl)-, 4-Morpholinyl- oder 1-Hexahydroazepinyl-Gruppe bilden}, weiterhin zusätzlich $R^2$ und $R^3$ zusammen mit dem Stickstoff-Atom einen 1-Azetidinyl-, 1-Pyrrolidinyl-, 1-Piperidinyl-, 4-Morpholinyl-, 1-(4-Alkylpiperazinyl)-, 1-(4-Alkoxyalkylpiperazinyl)-, 1-(4-Hydroxyalkylpiperazinyl), 1-(3-Hydroxyazetidinyl)-, 1-(3-Alkoxyazetidinyl)-, 1-(3-Hydroxypyrrolidinyl)-, 1-(3-Alkoxypyrrolidinyl)-, 1-(3- oder 4-Hydroxypiperidinyl)-, 1-(3- oder 4-Alkoxypiperidinyl)-, 1-(4-Oxopiperidinyl)- oder 1-(3-Oxopyrrolidinyl)-Ring bilden können; noch weiterhin zusätzlich dann, wenn $R^2$ Wasserstoff ist, $R^3$ $-CHR^7CO_2R^8$ sein kann, worin $R^7$ und $R^8$ unabhängig voneinander Wasserstoff, Alkyl, oder Aralkyl sind, |
| $R^9$ | Alkyl, Aralkyl, Aryl, Alkoxyalkyl, Aryloxyalkyl, Aralkoxyalkyl, Cycloalkylalkyl, Alkoxycarbonylalkyl, Cycloalkyl, 1-Adamantyl, Cycloalkoxyalkyl, Alkoxy, Aralkoxy, Cycloalkoxy, Aryloxy oder $-CHR^7NHR^8$ sein kann (worin $R^7$ und $R^8$ wie vorstehend definiert sind); |
| R' | ein $C_1$-$C_3$-Alkyl ist; |
| Z | X ist, wie vorstehend definiert, Amino, Alkylamino oder |

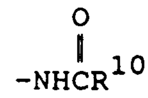

ist

(worin $R^{10}$ Wasserstoff, Alkyl oder Aryl ist); und

Alkyl und die Alkyl-Teile von Alkoxy und Aralkyl lineare oder verzweigte Kohlenstoff-Ketten mit 1 bis 6 Kohlenstoff-Atomen bezeichnen, und Aryl und die Aryl-Teile von Aralkyl unsubstituiertes Phenyl bezeichnen, sowie Phenyl, das monosubstituiert ist durch Alkyl, Hydroxy, Alkoxy, Halogen oder Trifluormethyl;

gekennzeichnet durch

(a) intramolekulare Kondensation einer Verbindung der Formel

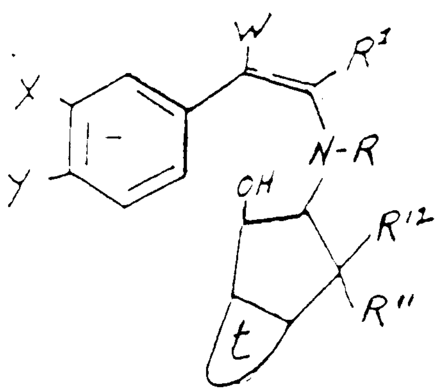

II

(b) Umsetzung einer Verbindung der Formel

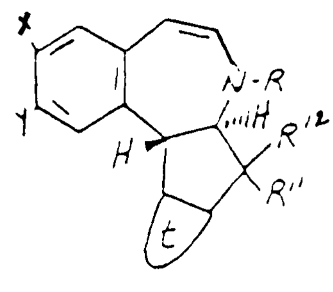

XI

mit einem Reduktionsmittel;

(c) Zusammenbringen einer Verbindung der Formel

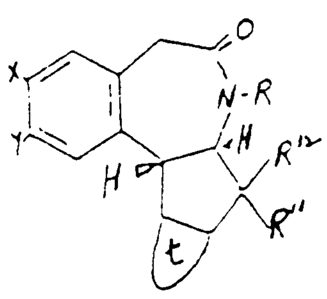

XIV

mit einem Reduktionsmittel; oder Umsetzung einer Verbindung der Formel XIII

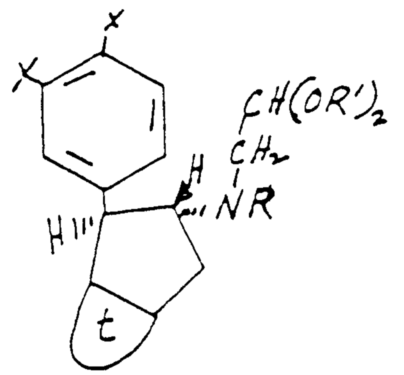

XIII

mit einer starken Säure.

**12.** Verfahren nach Anspruch 11, wobei die Substituenten wie in Anspruch 11 definiert sind, mit der Maßgabe, daß wenn X $OCH_3$ ist, Y OH ist, Z und $R^1$ beide H sind, R nicht $CH_3$ sein kann.

**13.** Verfahren zur Herstellung einer pharmazeutischen Zusammensetzung, umfassend das Vermischen einer Verbindung der Formel I mit einem pharmazeutisch annehmbaren Träger.

**Revendications**

**1.** Composé ayant la formule de structure trans isomère I, et ses sels acceptables en pharmacie,

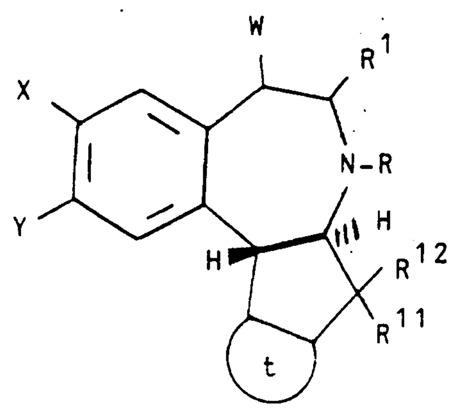

I

où :

R est hydrogène, alkyle; $-CH_2CH=CH_2$ ou $-CH_2$-◁ ;

$R^1$, $R^{11}$ et $R^{12}$ sont identiques ou différents et chacun est hydrogène ou alkyle ;

X est hydrogène, halo, alkyle, alkylthio, alkylsulfinyle, alkylsulfonyle, hydroxy, alcoxy ou trifluorométhyle ;

Y est hydrogène, hydroxy, alcoxy,

$$-O\overset{O}{\overset{\|}{C}}NR^2R^3, \quad -O\overset{O}{\overset{\|}{C}}-R^9, \quad -NR^1_2, \quad -NH\overset{O}{\overset{\|}{C}}R^1 \text{ ou } -O\overset{O}{\overset{\|}{P}}(OH)OR^1 \;;$$

où $R^1$ est tel que défini ci-dessus ;

W est hydrogène, hydroxy ou alcoxy ;

le noyau (t) représente un noyau thiophène fusionné ou benzène fusionné, ledit noyau benzène

fusionné étant facultativement substitué par un substituant Z tel que défini ci-dessous ;

$R^2$ et $R^3$ sont indépendamment hydrogène (à condition que tous deux ne soient pas hydrogène), alkyle, aralkyle, cycloalkyle, aryle, hydroxyalkyle ou alcoxyalkyle ;

de plus, lorsque l'un de $R^2$ et $R^3$ est tel que défini ci-dessus, l'autre peut être $-R^4NR^5R^6$ {où $R^4$ est alcanediyle, $R^5$ est hydrogène ou alkyle et $R^6$ est alkyle ou bien $R^5$ et $R^6$,avec l'atome d'azote, forment un groupe 1-azétidinyle, 1-pyrrolidinyle, 1-pipéridinyle, 1-(4-alkylpipérazinyle),4-morpholinyle ou 1-hexahydroazépinyle},

de plus encore, $R^2$ et $R^3$,avec l'atome d'azote, peuvent former un noyau 1-azétidinyle, 1-pyrrolidinyle, 1-pipéridinyle, 4-morpholinyle, 1-(4-alkylpipérazinyle), 1-(4-alcoxyalkylpipérazinyle), 1-(4-hydroxyalkylpipérazinyle), 1-(3-hydroxyazétidinyle), 1-(3-alcoxyazétidinyle), 1-(3-hydroxypyrrolidinyle), 1-(3-alcoxypyrrolidinyle), 1-(3 ou 4-hydroxypipéridinyle), 1-(3 ou 4-alcoxypipéridinyle), 1-(4-oxopipéridinyle) ou 1-(3-oxopyrrolidinyle) ;

de plus encore, quand $R^2$ est hydrogène, $R^3$ peut être $-CHR^7CO_2R^8$ , où $R^7$ et $R^8$ sont indépendamment hydrogène, alkyle ou aralkyle ;

$R^9$ est alkyle, aralkyle, aryle, alcoxyaryle, aryloxyalkyle, aralcoxyalkyle, cycloalkylalkyle, alcoxycarbonylalkyle, cycloalkyle, 1-adamantyle, cycloalcoxyalkyle, alcoxy, aralcoxy, cycloalcoxy, aryloxy ou $-CHR^7NHR^8$ { où $R^7$ et $R^8$ sont tels que définis ci-dessus};

Z est X comme défini ci-dessus, amino, alkylamino ou

$$-NH\overset{\overset{O}{\|}}{C}R^{10}$$

{ où $R^{10}$ est hydrogène, alkyle ou aryle } ; et

l'alkyle et les portions alkyles de l'alcoxy et de l'aralkyle représentent des chaînes droites ou ramifiées de carbone ayant 1 à 6 atomes de carbone et aryle et les portions aryles d'aralkyle représentent phényle non substitué et phényle monosubstitué par alkyle, hydroxy, alcoxy, halo ou trifluorométhyle.

**2.** Composé où les substituants sont tels que définis à la revendication 1, à condition que quand X est $OCH_3$ , Y est OH, Z et $R^1$ sont tous deux H, R ne puisse être $CH_3$.

**3.** Composé tel que défini à la revendication 1 ou à la revendication 2, où W, $R^{11}$ et $R^{12}$ sont tous hydrogène.

**4.** Composé tel que défini selon l'une quelconque des revendications 1-3 ci-dessus
où Y est hydroxy ;

$$-O-\overset{\overset{O}{\|}}{C}NR^2R^3$$

où $R^2$ et $R^3$ sont tous deux alkyle ou bien l'un de $R^2$ et $R^3$ est hydrogène et l'autre est alkyle ; $-NHR^1$ où $R^1$ est hydrogène ou méthyle ;

$$NH\overset{\overset{O}{\|}}{C}R^1$$

où $R^1$ est hydrogène ou méthyle ; ou bien

$$-O\overset{\overset{O}{\|}}{C}R^9$$

où $R^9$ est tel que défini à la revendication 1 ;
X est hydrogène, alkyle, halogène ou alcoxy ;
Z est hydrogène, halogène, alkyle, hydroxy ou alcoxy ;
R est méthyle ; et
$R^1$ est hydrogène ou méthyle.

**5.** Composé tel que défini selon l'une quelconque des revendications 1-4 ci-dessus où
Y est hydroxy, amino,

$$-O\overset{\overset{O}{\|}}{C}R^9, \quad O\overset{\overset{O}{\|}}{C}N(CH_3)_2$$

ou $-NHCH_3$ où $R^9$ est tel que défini à la revendication 1 ;
le noyau (t) est un noyau benzène fusionné ;
Z est hydrogène, halo, alkyle ou $-OR^1$ où $R^1$ est hydrogène ou alkyle ;
X est hydrogène, méthyle, méthoxy, chloro ou bromo;
R est méthyle ; et
$R^1$ est hydrogène.

**6.** Composé tel que défini à la revendication 1 qui est
(1) trans 5,6,7,7a,8,12b hexahydro-2-hydroxy-3-chloro-7-méthyl-benz[d]indéno-[2,1-b]azépine ou son sel acceptable en pharmacie ;
(2) (+)-trans 5,6,7,7a,8,12b-hexahydro-2-hydroxy-3-chloro-7-méthyl-benz[d]-indéno[2,1-b]azépine ou son sel acceptable en pharmacie ;
(3) (-)-trans 5,5,7,7a,8,12b-hexahydro-2-hydroxy-3-chloro-7-méthyl-benz[d]-indéno[2,1-b]azépine ou son sel acceptable en pharmacie ;
(4) trans-5,6,7,7a,8,12b-hexahydro-2-hydroxy-3-méthoxy-7-méthyl-benz[d]-indéno[2,1-b]azépine ou son sel acceptable en pharmacie ;
(5) trans - 5,6,7,7a,8,12b-hexahydro-2-amino-3-chloro-7-méthyl-benz[d]-indéno[2,1-b]azépine ou son sel acceptable en pharmacie ;
(6) trans - 5,6,7,7a,8,12b-hexahydro-2-hydroxy-7-méthyl-benz[d]-indéno[2,1-b]azépine ou son sel acceptable en pharmacie ;
(7) trans - 5,6,7,7a,8,12b-hexahydro-3,7-diméthyl-2-hydroxy-benz[d]-indéno[2,1-b]azépine ou son sel acceptable en pharmacie ;
(8) trans - 5,6,7,7a,8,12b-hexahydro-3-chloro-7-cyclopropylméthyl-2-hydroxy-benz[d]-indéno[2,1-b]-azépine ou son sel acceptable en pharmacie ;
(9) trans - 5,6,7,7a,8,12b-hexahydro-7-allyl-3-chloro-2-hydroxy-benz[d]-indéno[2,1-b]azépine ou son sel acceptable en pharmacie ;
(10) trans - 5,6,7,7a,8,12b-hexahydro-3-chloro-2-hydroxy-7,8,8-triméthyl-benz[d]-indéno[2,1-b]azépine ou son sel acceptable en pharmacie ; et
(11) trans - 3-chloro-5,6,7,7a,8,11b-hexahydro-7-méthyl-thiéno[2',3':4,5]cyclopenta[1,2-a][3]-benzazépin-2-ol ou son sel acceptable en pharmacie.

**7.** Composé de la revendication 1 qui est :
(1) trans - 5,6,7,7a,8,12b-hexahydro-2-hydroxy-3-chloro-7-méthyl-benz[d]indéno[-2,1-b]azépine ou son sel acceptable en pharmacie ;
(2) (±)-trans-5,6,7,7a,8,12b-hexahydro-2-hydroxy-3-chloro-7-méthyl-benz[d]-indéno[2,1-b]azépine ou son sel acceptable en pharmacie ;
(3) (-)-trans-5,6,7,7a,8,12b-hexahydro-2-hydroxy-3-chloro-7-méthyl-benz[d]-indéno[2,1-b]azépine ou son sel acceptable en pharmacie ;
(4) trans-5,6,7,7a,8,12b-hexahydro-2-hydroxy-3-méthoxy-7-méthyl-benz[d]-indéno[2,1-b]azépine ou son sel acceptable en pharmacie ;
(5) trans - 5,6,7,7a,8,12b-hexahydro-2-amino-3-chloro-7-méthyl-benz[d]-indéno[2,1-b]azépine ou son sel acceptable en pharmacie ;
(6) trans - 5,6,7,7a,8,12b-hexahydro-2-hydroxy-7-méthyl-benz[d]-indéno[2,1-b]azépine ou son sel acceptable en pharmacie ;

(7) trans - 5,6,7,7a,8,12b-hexahydro-3,7-diméthyl-2-hydroxy-benz[d]-indéno[2,1-b]azépine ou son sel acceptable en pharmacie ; et

(8) trans - 5,6,7,7a, 8,12b-hexahydro-2-hydroxy-3-chloro-7,8,8-tri-méthyl-benz[d]-indéno[2,1-b]-azépine.

**8.** Composition pharmaceutique qui comprend un composé tel que défini selon l'une des revendications 1-7 en combinaison avec un support acceptable en pharmacie.

**9.** Utilisation d'un composé selon l'une quelconque des revendications 1-6 pour la préparation d'une composition pharmaceutique utile dans le traitement des psychoses, dans le traitement de la douleur, dans le traitement de la dépression et/ou en tant que vasodilatateur rénal.

**10.** Composé de la formule II, XI, XIII ou XIV

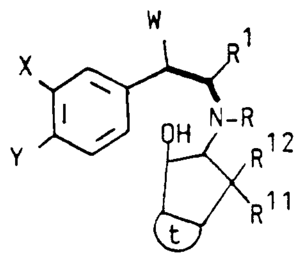

II

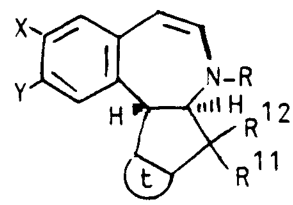

XI

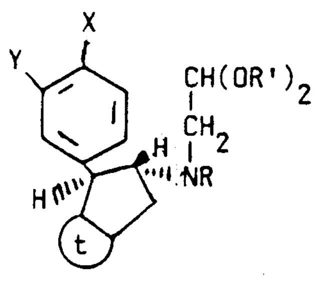

XIII

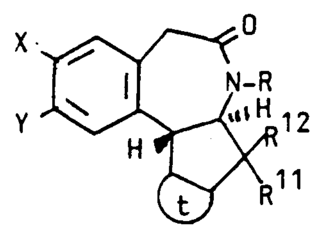

XIV

où

R est hydrogène, alkyle, $-CH_2CH=CH_2$ ou $-CH_2$-◁ ;

$R^1$, $R^{11}$ et $R^{12}$ sont identiques ou différents et chacun est hydrogène ou alkyle ;

X est hydrogène, halo, alkyle, alkylthio, alkylsulfinyle, alkylsulfonyle, hydroxy, alcoxy ou trifluorométhyle ;

Y est hydrogène, hydroxy, alcoxy,

$$-O\overset{O}{\overset{\|}{C}}NR^2R^3 \;,\quad -O\overset{O}{\overset{\|}{C}}-R^9,\quad -NR^1{}_2,\quad -NH\overset{O}{\overset{\|}{C}}R^1 \quad \text{ou} \quad -O\overset{O}{\overset{\|}{P}}(OH)OR^1 \;;$$

W est hydrogène, hydroxy ou alcoxy ;

le noyau (t) représente un noyau thiophène fusionné ou benzène fusionné, ledit noyau benzène fusionné étant facultativement substitué par un substituant Z tel que défini ci-dessous ;

$R^2$ et $R^3$ sont indépendamment hydrogène (à condition que tous deux ne soient pas hydrogène),

alkyle, aralkyle, cycloalkyle, aryle, hydroxyalkyle ou alcoxyalkyle ;

de plus, quand l'un de $R^2$ et $R^3$ est tel que défini ci-dessus, l'autre peut être $-R^4NR^5R^6$ { où $R^4$ est alkylène, $R^5$ est hydrogène ou alkyle et $R^6$ est alkyle ou $R^5$ et $R^6$ avec l'atome d'azote forment un groupe 1-azétidinyle, 1-pyrrolidinyle, 1-pipéridinyle, 1-(4-alkylpipérazinyle), 4-morpholinyle ou 1-hexahydroazépinyle},

de plus encore, $R^2$ et $R^3$, avec l'atome d'azote,peuvent former un noyau 1-azétidinyle, 1-pyrrolidinyle, 1-pipéridinyle, 4-morpholinyle, 1-(4-alkylpipérazinyle), 1-(4-alcoxyalkylpipérazinyle), 1-(4-hydroxyalkylpipérazinyle), 1-(3-hydroxyazétidinyle), 1-(3-alcoxyazétidinyle), 1-(3-hydroxypyrrolidinyle), 1-(3-alcoxypyrrolidinyle), 1-(3- ou 4-hydroxypipéridinyle), 1-(3- ou 4-alcoxypipéridinyle); 1-(4-oxopipéridinyle) ou 1-(4-oxopyrrolidinyle) ;

de plus encore, quand $R^2$ est hydrogène, $R^3$ peut de plus être $-CHR^7CO_2R^8$, ou $R^7$ et $R^8$ sont indépendamment hydrogène, alkyle ou aralkyle ;

$R^9$ est alkyle, aralkyle, aryle, alcoxyalkyle, aryloxyalkyle, aralcoxyalkyle, cycloalkylalkyle, alcoxycarbonylalkyle, cycloalkyle, 1-adamantyle, cycloalcoxyalkyle, alcoxy, aralcoxy, cycloalcoxy, aryloxy ou $-CHR^7NHR^8$, { où $R^7$ et $R^8$ sont tels que définis ci-dessus } ;

R' est alkyle $C_1$-$C_3$ ;

Z est X tel que défini ci-dessus, nitro, amino, alkylamino et

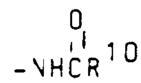

{ où $R^{10}$ est hydrogène, alkyle ou aryle } ; et

alkyle et les portions alkyles d'alcoxy et aralkyle représentent des chaînes droites ou ramifiées de carbone ayant 1 à 6 atomes de carbone et aryle et les portions aryles d'aralkyle représentent phényle non substitué et phényle monosubstitué par alkyle, hydroxy, alcoxy, halo ou trifluorométhyle.

**11.** procédé de production d'un composé ayant la formule de structure I

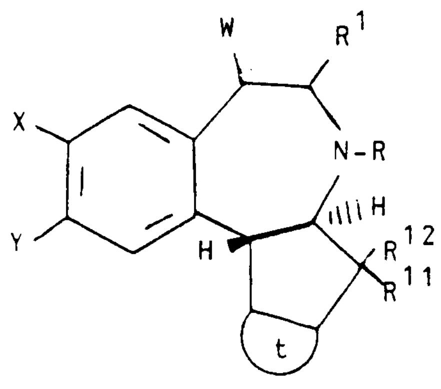

I

où

R est hydrogène, alkyle, $-CH_2CH=CH_2$ ou $-CH_2$-◁ ;

$R^1$, $R^{11}$ et $R^{12}$ sont identiques ou différents et chacun est hydrogène ou alkyle ;

X est hydrogène, halo, alkyle, alkylthio, alkylsulfinyle, alkylsulfonyle, hydroxy, alcoxy ou trifluorométhyle ;

Y est hydrogène, hydroxy, alcoxy,

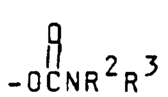, 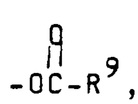, $-NR^1_2$, 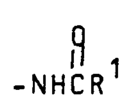 ou 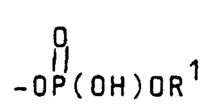 ;

W est hydrogène, hydroxy ou alcoxy ;

le noyau (t) représente un noyau thiophène fusionné ou benzène fusionné, ledit noyau benzène fusionné étant facultativement substitué par un substituant Z tel que défini ci-dessous ;

$R^2$ et $R^3$ sont indépendamment hydrogène (à condition que tous deux ne soient pas hydrogène), alkyle, aralkyle, cycloalkyle, aryle, hydroxyalkyle ou alcoxyalkyle ;

de plus, quand l'un de $R^2$ et $R^3$ est tel que défini ci-dessus, l'autre peut être $-R^4NR^5R^6$ { où $R^4$ est alcanediyle, $R^5$ est hydrogène ou alkyle et $R^6$ est alkyle ou bien $R^5$ et $R^6$ avec l'atome d'azote forment un groupe 1-azétidinyle, 1-pyrrolidinyle, 1-pipéridinyle, 1-(4-alkylpipérazinyle), 4-morpholinyle ou 1-hexahydroazépinyle }

de plus encore, $R^2$ et $R^3$ avec l'atome d'azote, peuvent former un noyau 1-azétidinyle, 1-pyrrolidinyle, 1-pipéridinyle, 4-morpholinyle, 1-(4-alkylpipérazinyle), 1-(4-alcoxyalkylpipérazinyle), 1-(4-hydroxyalkylpipérazinyle), 1-(3-hydroxyazétidinyle), 1-(3-alcoxyazétidinyle), 1-(3-hydroxypyrrolidinyle), 1-(3-alcoxypyrrolidinyle), 1-(3- ou 4-hydroxypipéridinyle), 1-(3- ou 4-alcoxypipéridinyle), 1-(4-oxopipéridinyle) ou 1-(3-oxopyrrolidinyle);

de plus encore, quand $R^2$ est hydrogène, $R^3$ peut être $-CHR^7CO_2R^8$, où $R^7$ et $R^8$ sont indépendamment hydrogène, alkyle ou aralkyle ;

$R^9$ est alkyle, aralkyle, aryle, alcoxyalkyle, aryloxyalkyle, aralcoxyalkyle, cycloalkylalkyle, alcoxycarbonylalkyle, cycloalkyle, 1-adamantyle, cycloalcoxyalkyle, alcoxy, aralcoxy, cycloalcoxy, aryloxy ou $-CHR^7NHR^8$ { où $R^7$ et $R^8$ sont tels que définis ci-dessus } ;

R' est un alkyle $C_1$-$C_3$ ;

Z est X tel que défini ci-dessus, amino, alkylamino ou

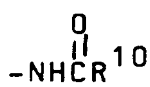

{ où $R^{10}$ est hydrogène, alkyle ou aryle } ; et

alkyle et les portions alkyles de l'alcoxy et de l'aralkyle représentent des chaînes droites ou ramifiées de carbone ayant 1 à 6 atomes de carbone et aryle et les portions aryles d'aralkyle représentent un phényle non substitué et un phényle monosubstitué par alkyle, hydroxy, alcoxy, halo ou trifluorométhyle ;

caractérisé par

(a) la condensation intramoléculaire d'un composé de la formule

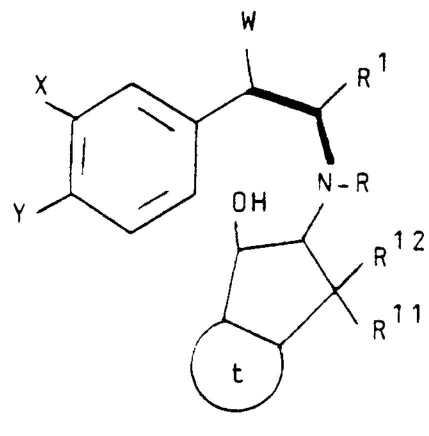

II

(b) par réaction d'un composé de la formule

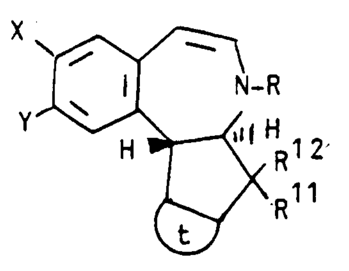

XI

avec un agent réducteur ;
(c) la mise en contact d'un composé de la formule

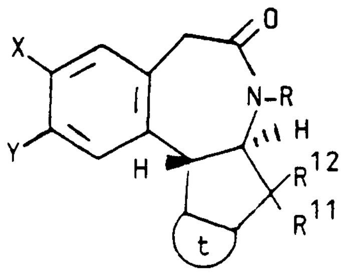

XIV

avec un agent réducteur ; ou la réaction d'un composé de la formule XIII

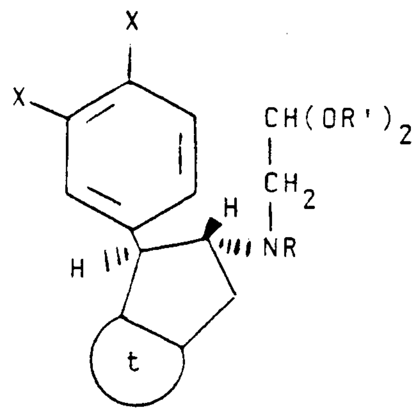

XIII

avec un acide fort.

**12.** Procédé selon la revendication 11, où les substituants sont tels que définis à la revendication 11, à condition que quand X est $OCH_3$ , Y est OH, Z et $R^1$ sont tous deux X, R ne puisse être $CH_3$.

**13.** Méthode de production d'une composition pharmaceutique comprenant le mélange d'un composé de formule I avec un support acceptable en pharmacie.